(19) **Europäisches Patentamt European Patent Office Office européen des brevets**

(11) **EP 4 293 626 A1**

(12) # EUROPÄISCHE PATENTANMELDUNG

(43) Veröffentlichungstag:
 **20.12.2023 Patentblatt 2023/51**

(21) Anmeldenummer: **22179088.4**

(22) Anmeldetag: **15.06.2022**

(51) Internationale Patentklassifikation (IPC):
 *G06T 15/08* (2011.01)

(52) Gemeinsame Patentklassifikation (CPC):
 **G06T 15/08;** G06T 2200/24; G06T 2210/41

(84) Benannte Vertragsstaaten:
 **AL AT BE BG CH CY CZ DE DK EE ES FI FR GB GR HR HU IE IS IT LI LT LU LV MC MK MT NL NO PL PT RO RS SE SI SK SM TR**
 Benannte Erstreckungsstaaten:
 **BA ME**
 Benannte Validierungsstaaten:
 **KH MA MD TN**

(71) Anmelder: **Siemens Healthcare GmbH 91052 Erlangen (DE)**

(72) Erfinder: **Krüger, Sebastian 91052 Erlangen (DE)**

(54) **VERFAHREN UND VORRICHTUNG ZUM BEREITSTELLEN EINES DREIDIMENSIONALEN MODELLS EINES OBJEKTS**

(57) Die Erfindung betrifft Verfahren und Vorrichtungen zum Bereitstellen eines dreidimensionalen Modells eines durch Volumendaten repräsentierten Objekts. Dabei wird ein Bildsynthesealgorithmus verwendet, der zur Visualisierung des dreidimensionalen Objekts durch Abbilden der Volumendaten auf Visualisierungspixel eines zweidimensionalen Visualisierungsbildes ausgebildet ist. Es wird ein Parametersatz zur Ansteuerung des Bildsynthesealgorithmus ermittelt, der geeignet ist, mit dem Bildsynthesealgorithmus einen Satz mehrerer verschiedener Visualisierungsbilder basierend auf dem Volumendatensatz zu erzeugen. Ferner wird der Satz mehrerer verschiedener Visualisierungsbilder durch Abbilden der Volumendaten mit dem Bildsynthesealgorithmus unter Verwendung des Parametersatzes erzeugt. Ferner wird das 3D-Modells basierend auf dem Satz mehrerer Visualisierungsbildes berechnet.

FIG 1

**EP 4 293 626 A1**

**Beschreibung**

Die Erfindung betrifft Verfahren und Vorrichtungen zum Erzeugen und Bereitstellen eines virtuellen dreidimensionalen Modells (3D-Modell) eines realen dreidimensionalen Objekts. Das 3D-Modell wird dabei zur Weitergabe an und Verwendung durch einen Nutzer bereitgestellt, der sich anhand des 3D-Modells verschiedene Ansichten des Objekts anzeigen lassen kann.

Die Modellierung, Rekonstruktion oder Visualisierung von dreidimensionalen Objekten hat einen weiten Anwendungsbereich in den Feldern Medizin (z. B. CT, PET), Physik (z. B. Elektronenstruktur großer Moleküle) oder Geophysik (Beschaffenheit und Lage von Erdschichten). Typischerweise wird das zu untersuchende Objekt bestrahlt (z.B. mittels elektromagnetischer Wellen oder Schallwellen), um seine Beschaffenheit zu untersuchen. Die gestreute Strahlung wird detektiert und aus den detektierten Werten werden Eigenschaften des Objekts ermittelt. Üblicherweise besteht das Ergebnis in einer physikalischen Größe (z. B. Dichte, Gewebetyp, Elastizität, Geschwindigkeit), deren Wert für das Objekt ermittelt wird. Dabei verwendet man in der Regel ein virtuelles Gitter, an dessen Gitterpunkten der Wert der Größe ermittelt wird. Diese Gitterpunkte werden üblicherweise als Voxel bezeichnet. Der Begriff "Voxel" ist ein aus den Begriffen "Volume" und "Pixel" gebildetes Synthesewort. Ein Voxel entspricht der Raumkoordinate eines Gitterpunktes, welchem der Wert einer Größe an diesem Ort zugeordnet ist. Dabei handelt es sich meist um eine physikalische Größe, die als skalares oder vektorielles Feld dargestellt werden kann, d.h. der Raumkoordinate ist der entsprechende Feldwert zugeordnet. Die so erhaltenen Daten des Objekts werden auch als Volumendaten bezeichnet. Durch Interpolation der Voxel kann man den Wert der Größe bzw. des Feldes an beliebigen Objektpunkten (d.h. an beliebigen Ortspunkten des untersuchten Objektes) erhalten.

Zur Visualisierung der Volumendaten wird üblicherweise aus den Voxeln eine dreidimensionale Darstellung des Objekts bzw. Körpers auf einer zweidimensionalen Darstellungsfläche (z.B. ein Bildschirm oder eine Scheibe bzw. Linse einer sog. "Augmented-Reality-Brille") erzeugt. Mit anderen Worten werden (in drei Dimensionen definierte) Voxel auf (in zwei Dimensionen definierte) Pixel eines zweidimensionalen Visualisierungsbildes abgebildet. Die Pixel des Visualisierungsbildes werden im Folgenden auch Visualisierungspixel genannt. Die Abbildung wird üblicherweise als Volume Rendering (Volumenwiedergabe) bezeichnet. Von der Durchführung der Volumenwiedergabe hängt es ab, wie in den Voxeln enthaltende Informationen mittels der Pixel wiedergegeben werden.

Eines der meistbenutzten Verfahren zur Volumenwiedergabe ist das sog. Ray-Casting (vgl. Levoy: "Display of Surfaces from Volume Data", IEEE Computer Graphics and Applications, Ausgabe 8, Nr. 3, Mai 1988, Seiten 29-37). Beim Ray-Casting werden simulierte Strahlen, die vom Auge eines imaginären Betrachters ausgehen, durch den untersuchten Körper bzw. das untersuchte Objekt gesendet. Entlang der Strahlen werden für Abtastpunkte RGBA-Werte aus den Voxeln bestimmt und zu Pixeln für ein zweidimensionales Bild mittels Alpha Compositing bzw. Alpha Blending vereinigt. Dabei stehen in dem Ausdruck RGBA die Buchstaben R, G und B für die Farbanteile rot, grün und blau, aus denen sich der Farbbeitrag des entsprechenden Abtastpunktes zusammensetzt. A steht für den ALPHA-Wert, der ein Maß für die Transparenz am Abtastpunkt darstellt. Die jeweilige Transparenz wird bei der Überlagerung von RGB-Werten an Abtastpunkten zu dem Pixel verwendet. Beleuchtungseffekte werden üblicherweise mittels eines Beleuchtungsmodells im Rahmen eines mit "Shading" bezeichneten Verfahrens berücksichtigt.

Ein weiteres Verfahren zur Volumenwiedergabe ist das sog. Path-Tracing oder Pfadverfolgungsverfahren (vgl. Kajiya: "The rendering equation", ACM SIGGRAPH Computer Graphics, Ausgabe 20, Nr. 4, August 1986, Seiten 143-150). Dabei werden pro Visualisierungspixel mehrere simulierte Strahlen in die Volumendaten geschossen, die dann mit dem Volumen wechselwirken, d.h. reflektiert, gebrochen oder absorbiert werden, wobei jedes Mal (außer im Falle der Absorption) mindestens ein zufälliger Strahl generiert wird. Jeder simulierte Strahl sucht sich so seinen Weg (path) durch die Volumendaten. Je mehr virtuelle Strahlen pro Visualisierungspixel verwendet werden, desto mehr nähert man sich dem idealen Bild an. Hierbei können insbesondere die in EP 3 178 068 B1 beschriebenen Methoden und Verfahren angewandt werden. Der Inhalt der EP 3 178 068 B1 ist hierin in seiner Gesamtheit durch Bezugnahme einbezogen.

Ein primäres technisches Hindernis bei der Implementierung eines Systems zur interaktiven Volumenwiedergabe ist das schnelle, effiziente und lokale Verarbeiten großer Datenmengen. Gerade auf Geräten mit eingeschränkter Rechen- bzw. Graphikleistung ist schnelles Volume Rendering eine Herausforderung. Beispiele für solche Geräte sind mobile Geräte wie Smartphones oder Tablets. Diese sind meist mit stromsparenden Prozessoren ("System on a Chip") ausgestattet, die im Vergleich zu modernen PCs vergleichsweise wenig Rechenleistung haben. Ferner ist die Portabilität eines Volumenwiedergabesystems oft eingeschränkt, da zur lokalen Verwendung sowohl der Volumenwiedergabe-Algorithmus als auch die Volumendaten portiert werden müssen. Dies ist aufgrund der Datenmengen und aus Gründen der Datensicherheit oft nicht möglich. Zudem ist das Auffinden geeigneter Parameter zur Erzeugung eines geeigneten Visualisierungsbildes oftmals nicht trivial und für den Endnutzer nur schwer zu überblicken, da der Endnutzer typischerweise kein Experte für Verfahren zur Volumenwiedergabe ist.

Dies hat zur Folge, dass eine interaktive Volumenwidergabe durch den Endnutzer häufig nur eingeschränkt oder gar nicht möglich ist. Im Regelfall werden dem Nutzer nur einzelne Visualisierungsbilder bereitgestellt. Gerade im medizinischen Umfeld ist es allerdings wichtig, dass ein Nutzer, etwa eine Ärztin oder ein Arzt, die Ansichten in der Volumenwidergabe frei verändern kann. Oft kann nur dadurch ein Verständnis des zugrundeliegenden Sachverhalts erreicht werden.

Es ist eine Aufgabe der Erfindung, in dieser Hinsicht verbesserte Verfahren und Vorrichtungen anzugeben, die insbesondere eine bessere Portabilität und Interaktivität von Volumenwiedergaben eines dreidimensionalen Objekts für einen Nutzer gewährleisten.

Diese und weitere Aufgabe werden mit einem Verfahren, einer Vorrichtung, einem Computerprogrammprodukt bzw. einem computerlesbaren Speichermedium gemäß dem Hauptanspruch und den nebengeordneten Ansprüchen gelöst. Vorteilhafte Weiterbildungen sind in den abhängigen Ansprüchen angegeben.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Gemäß einem Aspekt wird ein Computer-implementiertes Verfahren zum Bereitstellen eines 3D-Modells eines durch Volumendaten repräsentierten dreidimensionalen Objekts bereitgestellt. Das Verfahren weist mehrere Schritte auf. Ein Schritt ist auf ein Bereitstellen der Volumendaten gerichtet. Ein weiterer Schritt ist auf ein Bereitstellen eines Bildsynthesealgorithmus gerichtet, welcher Bildsynthesealgorithmus zur Visualisierung des dreidimensionalen Objekts durch Abbilden der Volumendaten auf Visualisierungspixel eines zweidimensionalen Visualisierungsbildes ausgebildet ist. Ein weiterer Schritt ist auf ein Ermitteln eines Parametersatzes zur Ansteuerung des Bildsynthesealgorithmus gerichtet, welcher Parametersatz geeignet ist, mit dem Bildsynthesealgorithmus einen Satz mehrerer verschiedener Visualisierungsbilder basierend auf dem Volumendatensatz zu erzeugen. Ein weiterer Schritt ist auf ein Erzeugen des Satzes mehrerer verschiedener Visualisierungsbilder durch Abbilden der Volumendaten mit dem Bildsynthesealgorithmus unter Verwendung des Parametersatzes gerichtet. Ein weiterer Schritt ist auf ein Berechnen des 3D-Modells basierend auf dem Satz mehrerer Visualisierungsbilder gerichtet. Ein weiterer Schritt ist auf ein Bereitstellen des 3D-Modells (für einen Nutzer) gerichtet.

Die Volumendaten können eine Mehrzahl von Voxeln aufweisen. Ein Voxel ("Volumenpixel" oder dreidimensionaler Pixel) ist ein Volumenelement, das einen Wert auf einem regulären Gitter im dreidimensionalen (3D) Raum darstellt. Voxel sind analog zu Pixeln, die zweidimensionale (2D) Bilddaten darstellen. Wie bei Pixeln enthalten die Voxel selbst typischerweise nicht ihre Position im Raum (ihre Koordinaten), sondern ihre Koordinaten werden abgeleitet basierend auf ihren Positionen relativ zu anderen Voxeln (also ihre Positionen in der Datenstruktur, die ein einzelnes Volumenbild bildet). Der Wert eines Voxels kann verschiedene physikalische Eigenschaften des dreidimensionalen Objekts repräsentieren, wie z.B. eine lokale Dichte. In Computertomographie Aufnahmen (CT-Scans) werden die Werte beispielsweise in Hounsfield-Einheiten ausgedrückt, welche die Opazität eines abgebildeten Materials in Bezug auf Röntgenstrahlen darstellen. Die Volumendaten beschreiben damit ein dreidimensionales Objekt in einem Objektvolumen. Insbesondere können die Volumendaten eine (insbesondere inhomogene) Dichte des dreidimensionalen Objekts im Objektvolumen angeben.

Die Volumendaten können insbesondere durch ein medizinisches Bildgebungsverfahren bereitgestellt werden. Die Bildgebungsverfahren können z.B. auf Röntgendurchleuchtung, Computertomographie (CT), Magnetresonanztomographie (MR), Ultraschall und/oder Positronen-Emissionstomographie (PET) beruhen. Entsprechend kann das dreidimensionale Objekt ein Körper oder Körperteil eines Patienten sein. Das dreidimensionale Objekt kann dabei ein oder mehrere Organe des Patienten umfassen.

Der Bildsynthesealgorithmus kann insbesondere als Computerprogrammprodukt aufgefasst werden, das zur Abbildung der Volumendaten auf eine zweidimensionale Projektionsfläche bzw. zur Volumenwiedergabe des dreidimensionalen Objekts bzw. zum Volume Rendering des dreidimensionalen Objekts ausgebildet ist. Die Projektionsfläche ist dabei durch das Visulisierungsbild gegeben. Der Bildsynthesealgorithmus kann Programmbestandteile in Form ein oder mehrerer Instruktionen für einen Prozessor zur Berechnung des Visualisierungsbildes aufweisen. Andere Begriffe für Bildsynthesealgorithmus sind beispielsweise "Renderer", "Renderalgorithmus" oder "Volumenrenderer". Bereitgestellt werden kann der Bildsynthesealgorithmus beispielsweise indem er in einer Speichereinrichtung vorgehalten wird oder in einen Arbeitsspeicher einer geeigneten Datenverarbeitungseinrichtung geladen wird oder allgemein zur Anwendung zur Verfügung gestellt wird.

Der Bildsynthesealgorithmus kann dabei verschiedene Verfahren zur Visualisierung eines Volumendatensatzes einzeln oder in Kombination implementieren. Beispielsweise kann der Bildsynthesealgorithmus ein Ray-Casting Modul und/oder ein Path-Tracing-Modul aufweisen. Der Bildsynthesealgorithmus bildet die Volumendaten auf die Visualisierungspixel ab.

Je nach den gewählten Einstellungen (Parameter) kann der Bildsynthesealgorithmus für ein Visualisierungsbild eine bestimmte Ansicht, Perspektive, Szenenillumination, Transferfunktion, Transparenz eines oder mehrerer Bereiche der Volumendaten, eine Schnittebene durch die Volumendaten, einen Interessenbereich innerhalb der Volumendaten, und/oder ein oder mehrere Zusatzinformationen zur Einblendung in das Visualisierungsbild realisieren. Eine Transferfunktion umfasst dabei Darstellungsparameter. Sie ordnen jedem Wert im dreidimensionalen Volumen eine bestimmte Farbe, Transparenz, Kontrast, Beleuchtung, Schärfe und dergleichen mehr zu. Allgemein ausgedrückt beeinflussen die Darstellungsparameter die Art der Darstellung von Objekten des entsprechenden Objekttyps in dem an den Nutzer ausgegebenen Visualisierungsbild.

Ein Visualisierungsbild ist das zweidimensionale Ergebnis des Volumenwiedergabeprozesses. Das Visualisierungsbild ist aus mehreren Visualisierungspixeln zusammengesetzt. Ebenso wie die Volumendaten können die Visualisierungsbilder abgespeichert werden.

Der Parametersatz stellt diejenigen Eingabeparameter für den Bildsynthesealgorithmus dar, auf deren Grundlage der Bildsynthesealgorithmus ein oder mehrere Visualisierungsbilder der Volumendaten erzeugen kann. Mit anderen Worten kann der Parametersatz als Steuerbefehlssatz für den Bildsynthesealgorithmus aufgefasst werden. In dem Parametersatz kann mit anderen Worten kodiert sein, welche Ansichten, Perspektiven, Szenenilluminationen, Transferfunktionen, Transparenzen, Schnittebenen usw. in den einzelnen Visualisierungsbildern jeweils realisiert sind.

Insbesondere kann der Parametersatz die Erzeugung von N verschiedenen Visualisierungsbildern vorgeben. N kann dabei größer oder gleich 6, bevorzugt größer als 10, weiter bevorzugt größer also 20 und weiter bevorzugt größer als 50 sein.

In der 3D-Computergrafik ist die 3D-Modellierung der Prozess der Entwicklung einer mathematischen und Koordinatenbasierten Darstellung einer beliebigen Oberfläche eines Objekts in drei Dimensionen. Dreidimensionale (3D-)Modelle stellen ein physisches Objekt z.B. dadurch dar, dass sie eine Sammlung von Punkten im 3D-Raum verwenden, die durch verschiedene geometrische Einheiten wie Dreiecke, Linien, gekrümmte Oberflächen usw. verbunden sind. Ihre Oberflächen können durch Texturabbildung (engl. Texture Mapping) weiter definiert werden. Insbesondere kann das 3D-Modell einen geringeren Speicherbedarf als die Volumendaten aufweisen.

Das 3D-Modell stellt also ein digitales bzw. virtuelles Modell des Objekts dar. Insbesondere stellt das 3D-Modell ein digitales bzw. virtuelles Ansichtsmodell des Objekts dar. Basierend auf dem 3D Modell können zweidimensionale Bilder durch 3D-Rendern erzeugt werden. Das Rendern eines 3D-Modells ist dabei oftmals einfacher als das Rendern von Volumendaten. Durch Manipulieren des 3D-Modells durch einen Nutzer kann das 3D-Modell deshalb stufenlos aus verschiedenen Ansichten betrachtet werden.

Bei der Berechnung des 3D-Modells erfolgt eine dreidimensionale Rekonstruktion des 3D-Modells aus den einzelnen Visualisierungsbildern, die insbesondere verschiedenen Ansichten des Objekts zeigen. Dabei stellen die Visualisierungsbilder nur diskrete Ansichten dar, über die bei der Berechnung des 3D-Modells interpoliert werden kann. Die Berechnung von 3D-Modellen aus Einzelbildern ist grundsätzlich bekannt.

Das 3D-Modell kann beispielsweise als Datensatz bereitgestellt werden. Insbesondere kann das 3D-Modell in einer Speichereinrichtung gespeichert und so bereitgestellt werden. Insbesondere kann das 3D-Modell zum Download bereitgestellt werden. Ferner kann das 3D-Modell durch Übermitteln beispielsweise über ein Internet und/oder ein Intranet bereitgestellt werden.

Durch das 3D-Modell basierend auf gerenderten Volumendaten wird ein Datensatz bereitgestellt, der gleichzeitig portabel und interaktiv visualisierbar ist. Durch den Schritt des Renderns der zugrundeliegenden Volumendaten mit dem Bildsynthesealgorithmus kann dabei eine hohe Bildqualität für Visualisierungen auf Grundlage des 3D-Modells gewährleistet werden. Gleichzeitig werden die Datenmenge und die Komplexität der Erzeugung von Visualisierungsbildern reduziert, was die Übertragbarkeit und die Anwenderfreundlichkeit für den Endnutzer verbessert. Mit anderen Worten werden die Volumendaten durch den Einsatz des Bildsynthesealgorithmus und das Berechnen des 3D-Modells im Hinblick auf deren dreidimensionale Visualisierung vorverarbeitet, komprimiert, optimiert und codiert.

Gemäß einem Aspekt erfolgt der Schritt des Berechnens des 3D-Modells durch Photogrammetrie.

In beispielhaften Ausführungsformen umfasst die Berechnung durch Photogrammetrie ein Extrahieren dreidimensionaler Messungen aus zweidimensionalen Daten (d.h. den Visualisierungsbildern). Beispielsweise kann der Abstand zwischen zwei Punkten, die auf einer Ebene parallel zur Bildebene liegen, durch Messen ihres Abstands in dem Visualisierungsbild bestimmt werden, da der Maßstab bekannt ist. Weiterhin können Farbbereiche und/oder Farbwerte, die solche Größen wie Albedo, Spiegelreflexion, Metallizität oder Umgebungsverdeckung zum Zweck der Berechnung des 3D-Modells extrahiert werden.

Durch Photogrammetrie kann das 3D-Modell auf einfache und zuverlässige Weise aus den Visualisierungsbildern erzeugt werden.

Gemäß einem Aspekt weist der Satz mehrere verschiedene Visualisierungsbilder auf, die jeweils verschiedene Ansichten des dreidimensionalen Objekts darstellen.

Durch die Bereitstellung verschiedener Ansichten kann das 3D-Modell effizient erzeugt werden.

Gemäß einem Aspekt ist das 3D-Modell als Oberflächenmodell implementiert.

Oberflächenmodell heißt mit anderen Worten, dass die Oberflächen des 3D-Modells mit Texturen und Farben belegt und nicht etwa transparent sind. Eine solche Ausgestaltung eignet sich für die anschließende Weitergabe des 3D-Modells und die weitere Verwendung durch einen Endnutzer, da sich das Modell so auf wesentliche Daten beschränkt, die zudem durch den Endnutzer einfach interpretiert werden können.

Gemäß einem Aspekt umfasst das Verfahren ferner einen Schritt des Bereitstellens eines Anforderungsprofils für das zu erstellende 3D-Modell, wobei das Anforderungsprofil eine oder mehrere Eigenschaften des zu erstellenden 3D-Modells angibt, wobei im Schritt des Ermittelns des Parametersatzes der Parametersatz zusätzlich basierend auf dem Anforderungsprofil ermittelt wird.

Beispielsweise kann das Anforderungsprofil vorbestimmt sein. Ferner kann das Bereitstellen des Anforderungsprofils ein Auswählen aus mehreren verschiedenen Auswahl-Anforderungsprofilen umfassen. Dabei können die Auswahl-Anforderungsprofile beispielsweise jeweils verschiedene Verwendungszwecke des 3D-Modells betreffen. Das Anforderungsprofil kann übergeordnete Eigenschaften des 3D-Modells betreffen, die unabhängig von dem konkreten Datensatz sind, wie etwa ein Datenvolumen, die im 3D-Modell verfügbaren Ansichten des Objekts, oder eine Auflösung des 3D-Modells. Das Anforderungsprofil kann ferner von den Volumendaten abhängen. So erfordern gering aufgelöste Volumendaten oftmals andere Visualisierungsbilder zur Erzeugung des 3D-Modells als höher aufgelöste Volumendaten. Folglich kann das Anforderungsprofil auch basierend auf den Volumendaten bereitgestellt werden.

Wird der Parametersatz basierend auf dem Anforderungsprofil bestimmt, können gezielt geeignete Parameter zur Ansteuerung des Bildsynthesealgorithmus bereitgestellt werden. Dadurch können automatisch solche Visualisierungsbilder erzeugt werden, die zu einem 3D-Modell mit den gewünschten Eigenschaften führen.

Gemäß einem Aspekt weist der Parametersatz eine erste Parametrierung und eine zweite Parametrierung auf. Die erste Parametrierung betrifft die (übergeordneten) Abbildungseigenschaften der Volumendaten (bzw. ist dazu ausgebildet, diese festzulegen). Die zweite Parametrierung ist zur Erzeugung mehrerer verschiedener Ansichten des dreidimensionalen Objekts mit der gleichen ersten Parametrierung ausgebildet, um so die mehreren verschiedenen Visualisierungsbilder des Satzes zu erzeugen.

Durch die Aufteilung in erste und zweite Parametrierung können verschiedene Visualisierungsbilder erzeugt werden, die zwar unterschiedliche Ansichten des Objekts zeigen, aber die gleichen globalen Abbildungseigenschaften, wie Szenenillumination, Farbe, Transparenz, Kontrast, Beleuchtung, Schärfe usw. aufweisen. Dadurch kann ein kohärenter Satz an Visualisierungsbildern erzeugt werden, was die Berechnung des 3D-Modells erleichtert.

Gemäß einem Aspekt umfasst die erste Parametrierung eine Transferfunktion zur Abbildung der Volumendaten auf das Visualisierungsbild und/oder eine Segmentierung des dreidimensionalen Objekts und/oder eine Clippingmaske.

Eine Transferfunktion umfasst dabei Darstellungsparameter. Sie ordnen jedem Wert in den Volumendaten eine bestimmte Farbe, Transparenz, Kontrast, Beleuchtung, Schärfe und dergleichen mehr zu. Allgemein ausgedrückt beeinflussen die Darstellungsparameter die Art der Darstellung von Objekten des entsprechenden Objekttyps in dem an den Nutzer ausgegebenen Visualisierungsbild.

Eine Clippingmaske kann beispielsweise als Clipping-Ebene oder andersartige Clipping-Fläche implementiert sein, mit der einzelne Bestandteile des Objekts weggeschnitten und somit von der Volumenwiedergabe durch den Bildsynthesealgorithmus ausgeschlossen werden können. Beispielsweise kann so in ein Objektvolumen geblickt werden, wenn eine Außenfläche durch eine Clippingmaske weggeschnitten ist.

Eine Segmentierung kann eine Identifizierung wenigstens eines Bereichs in den Volumendaten umfassen, wodurch dieser Bereich in der Volumenwiedergabe anders behandelt werden kann als die restlichen Volumendaten außerhalb des Bereichs.

Durch die Verwendung einer einheitlichen Transferfunktion bzw. Segmentierung bzw. Clippingmaske kann nicht nur ein einheitliches Erscheinungsbild der Visualisierungsbilder gewährleistet werden, sondern es können auch Segmentierungen und/oder Clipping-Effekte in dem 3D-Modell umgesetzt werden.

Gemäß einem Aspekt umfasst das Verfahren einen Schritt eines Bereitstellens eine Präferenz des Nutzers zur Volumenwiedergabe, wobei der Parametersatz basierend auf der Präferenz des Nutzers erzeugt wird.

Die Präferenz kann beispielsweise bevorzugte Abbildungseigenschaften eines Endnutzers umfassen. Dies kann beispielsweise eine bevorzugte Szenenillumination, eine bestimmte Farbe, Transparenz, Kontrast, Beleuchtung, Schärfe, Clipping-Fläche, Segmentierung und dergleichen mehr umfassen. Insbesondere kann eine Präferenz eine bevorzugte Transferfunktion und/oder eine bevorzugte erste Parametrierung umfassen.

Durch die Berücksichtigung der Nutzerpräferenz können nicht nur individuelle Vorlieben eines Nutzers berücksichtigt werden, sondern der Nutzer kann auch mitentscheiden, welche Partien des Objekts bei der Erzeugung des 3D-Modells zu berücksichtigen sind. So kann er durch die Präferenz z.B. eine Clipping-Ebene setzen, um in das Innere des Objekts zu blicken.

Gemäß einem Aspekt umfasst das Bereitstellen der Präferenz eine Bereitstellung mehrerer verschiedener Beispiel-Visualisierungsbilder für den Nutzer zur Auswahl, wobei jedes der Beispiel-Visualisierungsbilder mit unterschiedlichen Abbildungseigenschaften bzw. einer unterschiedlichen ersten Parametrierung erzeugt wurde, sowie ein Empfangen einer Auswahl eines der Beispiel-Visualisierungsbilder durch den Nutzer sowie ein Bestimmen der Präferenz basierend auf den Abbildungs- eigenschaften und/oder der ersten Parametrierung des ausgewählten Beispiel-Visualisierungsbilds. Insbesondere können die Beispiel-Visualisierungsbilder ebenfalls basierend auf den Volumendaten unter Verwendung des Bildsynthesealgorithmus erzeugt werden.

Dadurch kann der Nutzer auf einfache Weise eine Präferenz festlegen, ohne sich mit den komplexen Einstellmöglichkeiten des Bildsynthesealgorithmus befassen zu müssen.

Gemäß einem Aspekt erfolgen die Schritte des Bereitstellens der Volumendaten, des Bereitstellens des Bildsynthesealgorithmus, des Ermittelns des Parametersatzes, des Erzeugens des Satzes und des Berechnens des 3D-Modells in einer ersten Recheneinrichtung. Im Schritt des Bereitstellens wird das 3D-Modell einer zweiten Recheneinrichtung bereitgestellt, welche zweiten Recheneinrichtung von der ersten Recheneinrichtung verschieden ist, wobei die zweite Recheneinrichtung insbesondere ein tragbares Nutzerendgerät umfasst.

Die erste Recheneinrichtung kann als zentrale oder dezentrale Recheneinrichtung ausgebildet sein. Die erste Recheneinrichtung kann insbesondere als lokaler oder Cloud-basierter Verarbeitungsserver implementiert sein. Die erste Recheneinrichtung kann einen oder mehrere Prozessoren aufweisen. Die Prozessoren können als zentrale Verarbeitungseinheit (ein englischer Fachausdruck hierfür ist "central processing unit", kurz CPU) und/oder als Grafikprozessor (ein englischer Fachausdruck hierfür ist "graphics processing unit", kurz GPU) ausgebildet sein.

Die zweite Recheneinrichtung kann als Client oder Nutzerclient ausgebildet sein. Die zweite Recheneinrichtung kann eine Nutzerschnittstelle aufweisen, über die ein Endnutzer mit dem 3D-Modell interagieren und sich verschiedene Ansichten des 3D-Modells anzeigen lassen kann. Die Nutzerschnittstelle kann hierfür eine Eingabevorrichtung, wie etwa einen berührungsempfindlichen Bildschirm oder eine Computertastatur, und eine Ausgabevorrichtung, wie einen Bildschirm aufweisen. Die zweite Recheneinrichtung kann einen oder mehrere Prozessoren aufweisen. Die Prozessoren können als zentrale Verarbeitungseinheit (ein englischer Fachausdruck hierfür ist "central processing unit", kurz CPU) und/oder als Grafikprozessor (ein englischer Fachausdruck hierfür ist "graphics processing unit", kurz GPU) ausgebildet sein. Die zweite Recheneinrichtung kann als sog. ein-Chip-System (ein englischer Fachausdruck hierfür ist "system-on-a-chip", kurz SoP) ausgebildet sein, das alle Funktionen eines Geräts steuert. Die Recheneinrichtung kann insbesondere als tragbares Nutzerendgerät wie etwa ein Laptop, Tablet oder ein Smartphone ausgebildet sein.

Mit anderen Worten kann die vergleichsweise aufwendige Berechnung des 3D-Modells einschließlich des Renderns der Visualisierungsbilder in einer leistungsstarken Recheneinrichtung erfolgen. Anschließend kann das 3D-Modell einer Recheneinrichtung bereitgestellt werden, die über weniger Rechenleistung verfügt. An der zweiten Recheneinrichtung muss lediglich eine Visualisierung für den Endnutzer basierend auf dem 3D-Modell erfolgen. Dies ist aufgrund des geringeren Datenumfangs einfacher als eine Visualisierung basierend auf den Volumendaten. Somit werden die rechenaufwendigen und in der Aussteuerung durch den Nutzer komplizierten Schritte off-line in der ersten Recheneinrichtung durchgeführt. Dadurch können die zur Visualisierung der Volumendaten notwendigen Informationen einfach übertragen werden und in der zweiten Recheneinrichtung wird eine einfache und interaktive Visualisierung durch den Nutzer möglich.

Gemäß einem Aspekt umfasst die Bereitstellung eine Übertragung des 3D-Modells von der ersten zur zweiten Recheneinrichtung über das Internet.

Durch die Übertragung über das Internet kann eine flexible Bereitstellung des 3D-Modells und somit ein verbesserter Zugriff erfolgen.

Gemäß einem Aspekt implementiert der Bildsynthesealgorithmus ein path-tracing- oder ray-casting-Verfahren.

Durch derartige Verfahren können besonders wirklichkeitsnahe Visualisierungen erzeugt werden, was den Nutzen des Gesamtverfahrens erhöht. Die genannten Verfahren sind zwar in der Anwendung komplex; durch die erfindungsgemäße Umsetzung in ein 3D-Modell können die genannten Verfahren durch einen Endnutzer jedoch einfach verwendet werden und intrinsisch eine gute Visualisierung liefern.

Gemäß einem Aspekt sind die Volumendaten durch ein medizinisches Bildgebungsverfahren erzeugt und repräsentieren ein oder mehrere Organe eines Patienten. Ferner umfasst das Verfahren einen Schritt eines Bereitstellens einer dem Patienten zugeordneten Patienteninformation, wobei der Parametersatz basierend auf der Patienteninformation erzeugt wird.

Die Patienteninformation kann eine medizinische Information umfassen, die dem Patienten und oder den Volumendaten des Patienten zugeordnet ist. Die Patienteninformation kann beispielsweise ein oder mehrere Körperteile und/oder ein oder mehrere Organe des Patienten angeben bzw. betreffen bzw. enthalten. Die Patienteninformation kann ferner beispielsweise einen oder mehrere Befunde des Patienten bzw. eines Körperteils oder Organs des Patienten angeben bzw. betreffen bzw. enthalten. Die Patienteninformation kann mit anderen Worten anzeigen, welche Aspekte der Volumendaten für den jeweiligen Patienten besonders relevant sind und somit bei der Erzeugung der Visualisierungsbilder entsprechend berücksichtigt und/oder hervorgehoben werden können.

Durch die Berücksichtigung der Patienteninformation können der Parametersatz und damit die Visualisierungsbilder spezifisch auf den für den Patienten relevanten Anwendungsfall angepasst werden. Somit kann sichergestellt werden, dass relevante Partien der Volumendaten auch in dem 3D-Modell enthalten sind.

Gemäß einem Aspekt kann die Patienteninformation ein strukturiertes oder unstrukturiertes Dokument oder Datum umfassen, das ein oder mehrere Textelemente enthält.

Gemäß einem Aspekt umfassen die Patientendaten einen medizinischen Befundsbericht des Patienten, und/oder eine elektronische Patientenakte des Patienten, und/oder eine Befundungsaufgabe zur Erstellung eines Befundes für den Patienten durch einen Nutzer.

In den genannten Patienteninformationen können insbesondere Körperteile und/oder Organe des Patienten bezeichnet sein, die von den Volumendaten repräsentiert sind. Die Nennung in den genannten Patienteninformationen kann entsprechend andeuten, dass diese Körperteile und/oder Organe für das 3D-Modell relevant und entsprechend zu berücksichtigen sind.

Gemäß einem Aspekt wird das 3D-Modell im Schritt des Bereitstellens als weitere Patienteninformation und insbesondere als Teil eines (elektronischen) medizinischen Befundsberichts für den Patienten bereitgestellt.

Dadurch wird das 3D-Modell dem Patienten zugeordnet und archiviert und ist für die spätere Nutzung verfügbar.

Gemäß einem Aspekt umfasst das Verfahren ferner einen Schritt eines Bestimmens wenigstens eines Auswahl-Organes zur Darstellung in dem 3D-Modell basierend auf der Patienteninformation, sowie einen Schritt eines Erzeugens einer Segmentierungsmaske für die Volumendaten zur Segmentierung des wenigstens einen Auswahl-Organs, wobei im Schritt des Erzeugens die mehreren verschiedenen Visualisierungsbilder zusätzlich basierend auf der Segmentierungsmaske erzeugt werden.

Insbesondere kann aus der Patienteninformation eine Angabe des wenigstens einen Auswahl-Organs extrahiert werden. Hierfür können beispielsweise bekannte Computerlinguistikalgorithmen verwendet werden, die dazu ausgebildet sind, in der Patienteninformation enthaltene Textelemente zu erkennen und diesen eine Bedeutung beizumessen.

Mit anderen Worten kann durch eine Auswertung der Patienteninformation automatisch eine Segmentierung der Volumendaten erfolgen. Dadurch kann eine auf den medizinischen Anwendungsfall zielgerichtete Berechnung des 3D-Modells erfolgen.

Gemäß einem Aspekt weist die Patienteninformation eine auf das dreidimensionale Objekt bezogene Annotation auf, wobei im Schritt des Erzeugens die Annotation in das 3D-Modell eingefügt wird.

Die Annotation kann sich beispielsweise auf wenigstens ein Körperteil und/oder Organ des Patienten beziehen, die in den Volumendaten repräsentiert sind. Die Annotation kann beispielsweise eine Messung und/oder eine Notiz umfassen, die für die Volumendaten angelegt wurde. Beispielsweise kann die Annotation in räumlicher Nähe zum jeweiligen Körperteil und/oder Organ in das 3D-Modell eingefügt werden. Alternativ kann die Annotation in die Visualisierungsbilder eingefügt werden.

Dadurch kann das 3D-Modell mit zusätzlicher Information angereichert werden, die dann zusammen mit dem 3D-Modell bereitgestellt wird.

Gemäß einem Aspekt wird das 3D-Modell in einer Viewing-Applikation bereitgestellt werden, die es dem Nutzer ermöglicht, verschiedene Ansichten des 3D-Modells auszuwählen und anzusehen.

Beispielsweise kann die Viewing-Applikation dazu ausgebildet sein, zweidimensionale Visualisierungsbilder des 3D-Modells zu berechnen. Ferner kann die Viewing-Applikation dazu ausgebildet sein, eine auf die Auswahl einer Ansicht des 3D-Modells gerichtete Nutzereingabe zu empfangen und auf Grundlage der Nutzereingabe die ausgewählte Ansicht als Visualisierungsbild des 3D-Modells bereitzustellen. Insbesondere kann die Viewing-Applikation dazu ausgebildet sein, in der zweiten Recheneinrichtung abzulaufen.

Durch die Bereitstellung als Viewing-Applikation wird eine einfache Weitergabe des 3D-Modells mit einer interaktiven Bedienung ermöglicht.

Gemäß einem Aspekt ist die Viewing-Applikation als Web-Applikation implementiert. Dies hat den Vorteil eines einfachen Zugangs und einer leichtgewichtigen Implementierung der Viewing-Applikation.

Gemäß einem Aspekt kann im Schritt des Bereitstellens des 3D-Modells das 3D-Modell zum Download in einer Speichereinrichtung bereitgestellt werden.

Beispielsweise kann die Speichereinrichtung als Cloud-Speicher ausgebildet sein. Alternativ kann die Speichereinrichtung als lokaler Speicher ausgebildet sein. Zum Download bereitgestellt kann insbesondere heißen, dass das 3D-Modell für einen Nutzer in der Speichereinrichtung zugänglich gemacht wird.

Beispielsweise kann der Schritt des Bereitstellens ein Erzeugen eines Links zum direkten Zugriff auf das in der Speichereinrichtung zum Download bereitgestellte 3D-Modell und ein Bereitstellen des Links umfassen. Insbesondere kann der Link in der Viewing-Applikation bereitgestellt werden bzw. über diese abrufbar sein. Dadurch kann sowohl eine einfache Weitergabe des 3D-Modells als auch eine gute Zugänglichkeit für einen Nutzer erreicht werden.

Gemäß einem Aspekt umfasst das Verfahren ferner einen Schritt des Bereitstellens einer trainierten Funktion, die dazu ausgebildet ist, basierend auf Volumendaten (und optional einer Patienteninformation und/oder eines Anforderungsprofils und/oder einer Nutzerpräferenz) einen Parametersatz zur Ansteuerung eines Bildsynthesealgorithmus zu ermitteln, welcher Parametersatzes geeignet ist, mit dem Bildsynthesealgorithmus einen Satz mehrerer verschiedener Visualisierungsbilder basierend auf dem Volumendatensatz zu erzeugen, wobei im Schritt des Ermittelns des Parametersatzes der Parametersatzes durch Anwenden der trainierten Funktion (auf die Volumendaten und optional einer Patienteninformation und/oder eines Anforderungsprofils und/oder einer Nutzerpräferenz) ermittelt wird.

Eine trainierte Funktion bildet allgemein Eingabedaten auf Ausgabedaten ab. Hierbei können die Ausgabedaten insbesondere weiterhin von einem oder mehreren Parametern der trainierten Funktion abhängen. Der eine oder die mehreren Parameter der trainierten Funktion können durch ein Training bestimmt und/oder angepasst werden. Das Bestimmen und/oder das Anpassen des einen Parameters oder der mehreren Parameter der trainierten Funktion kann insbesondere auf einem Paar aus Trainingseingabedaten und zugehörigen Trainingsausgabedaten basieren, wobei die trainierte Funktion zur Erzeugung von Trainingsabbildungsdaten auf die Trainingseingabedaten angewendet wird. Insbesondere können das Bestimmen und/oder das Anpassen auf einem Vergleich der Trainingsabbildungsdaten und der Trainingsausgabedaten basieren. Im Allgemeinen wird auch eine trainierbare Funktion, d.h. eine Funktion mit noch nicht angepassten Parametern, als trainierte Funktion bezeichnet.

Andere Begriffe für trainierte Funktion sind trainierte Abbildungsvorschrift, Abbildungsvorschrift mit trainierten Parametern, Funktion mit trainierten Parametern, Algorithmus basierend auf künstlicher Intelligenz, Algorithmus des maschinellen Lernens. Ein Beispiel für eine trainierte Funktion ist ein künstliches neuronales Netzwerk. Anstatt des Begriffs "neuronales Netzwerk" kann auch der Begriff "neuronales Netz" verwendet werden. Ein neuronales Netzwerk ist im Grunde genommen wie ein biologisches neuronales Netz wie etwa ein menschliches Gehirn aufgebaut. Insbesondere umfasst ein künstliches neuronales Netzwerk eine Eingabeschicht und eine Ausgabeschicht. Es kann ferner mehrere Schichten zwischen Eingabe- und Ausgabeschicht umfassen. Jede Schicht umfasst mindestens einen, vorzugsweise mehrere Knoten. Jeder Knoten kann als biologische Verarbeitungseinheit verstanden werden, z. B. als Neuron. Mit anderen Worten entspricht jedes Neuron einer Operation, die auf Eingabedaten angewendet wird. Knoten einer Schicht können durch Kanten oder Verbindungen mit Knoten anderer Schichten verbunden sein, insbesondere durch gerichtete Kanten oder Verbindungen. Diese Kanten oder Verbindungen definieren den Datenfluss zwischen den Knoten des Netzwerks. Die Kanten oder Verbindungen sind mit einem Parameter assoziiert, der häufig als "Gewicht" oder "Kantengewicht" bezeichnet wird. Dieser Parameter kann die Wichtigkeit der Ausgabe eines ersten Knotens für die Eingabe eines zweiten Knotens regulieren, wobei der erste Knoten und der zweite Knoten durch eine Kante verbunden sind.

Insbesondere kann ein neuronales Netzwerk trainiert werden. Insbesondere wird das Training eines neuronalen Netzwerks basierend auf den Trainingseingabedaten und zugehörigen den Trainingsausgabedaten gemäß einer "überwachten" Lerntechnik (ein englischer Fachbegriff ist "supervised learning") durchgeführt, wobei die bekannten Trainingseingabedaten in das neuronale Netzwerk eingegeben und der die vom Netzwerk generierten Ausgabedaten mit den zugehörigen Trainingsausgabedaten verglichen werden. Das künstliche neuronale Netzwerk lernt und passt die Kantengewichte für die einzelnen Knoten unabhängig an, solange die Ausgabedaten der letzten Netzwerkschicht den Trainingsausgabedaten nicht ausreichend entsprechen.

Insbesondere kann eine trainierte Funktion auch ein tiefes künstliches neuronales Netzwerk sein (englischer Fachbegriff sind "deep neural network" oder "deep artificial neural network"). Gemäß einigen Implementierungen weist die trainierte Funktion ein neuronales Netzwerk und insbesondere ein faltendes neuronales Netzwerk auf. Ein englischer Fachbegriff für faltendes neuronales Netzwerk ist convolutional neural network. Insbesondere kann das faltende neuronale Netzwerk als tiefes faltendes neuronales Netzwerk ausgebildet sein (ein englischer Fachbegriff ist "deep convolutional neural network"). Das neuronale Netzwerk weist dabei ein oder mehrere Faltungsschichten (ein englischer Fachbegriff ist "convolutional layer") und ein oder mehrere Entfaltungsschichten (ein englischer Fachbegriff ist "deconvolutional layer") auf. Insbesondere kann das neuronale Netzwerk eine Sammelschicht umfassen (ein englischer Fachbegriff ist "pooling layer"). Durch die Verwendung von Faltungsschichten und/oder Entfaltungsschichten kann ein neuronales Netzwerk besonders effizient zur Ableitung eines Parametersatzes eingesetzt werden, da trotz vieler Verbindungen zwischen Knotenschichten nur wenige Kantengewichte (nämlich die den Werten des Faltungskerns entsprechenden Kantengewichte) bestimmt werden müssen. Bei einer gleichen Zahl von Trainingsdaten kann damit auch die Genauigkeit des neuronalen Netzwerks verbessert werden. Insbesondere hat sich gezeigt, dass faltende neuronale Netzwerke Volumendaten als Eingangsdaten gut verarbeiten können.

Beispielsweise kann ein Datensatz zum Trainieren der trainierten Funktion Trainingseingabedaten und Trainingsausgabedaten umfassen. Trainingseingabedaten umfassen Volumendaten und ggf. eine Patienteninformation und/oder ein Anforderungsprofil und/oder eine Nutzerpräferenz. Trainingsausgabedaten umfassen einen (verifizierten) Trainings-Parametersatz zur Eingabe in den Bildsynthesealgorithmus und zum Erzeugen mehrerer Visualisierungsbilder. Der Trainings-Parametersatz kann beispielsweise von einer Expertenperson bereitgestellt werden, die sowohl mit Bildsynthesealgorithmen als auch mit den Anforderungen an die Visualisierungsbilder zur Berechnung des 3D-Modells vertraut ist.

Durch die Verwendung einer trainierten Funktion kann der Parametersatz effizient automatisch erzeugt werden. Im Vergleich zu einer regelbasierten Erzeugung hat die Verwendung einer trainierten Funktion den Vorteil, dass die trainierte Funktion durch das Training in die Lage versetzt wird, eine dynamische Anpassung an verschiedene Sachverhalte zu schaffen.

Gemäß einem Aspekt wird ein Verfahren zum Bereitstellen einer trainierten Funktion bereitgestellt, die dazu ausgebildet ist, einen Parametersatz bereitzustellen, mit dem ein Bildsynthesealgorithmus zur Erzeugung eines 3D-Modells angesteuert werden kann. Das Verfahren weist mehrere Schritte auf. Ein erster Schritt ist auf ein Bereitstellen von Trainingseingangsdaten gerichtet, wobei die Trainingseingangsdaten ein dreidimensionales Objekt repräsentierende Volumendaten (und optional eine Patienteninformation und/oder ein Anforderungsprofil und/oder eine Nutzerpräferenz) aufweisen. Ein weiterer Schritt ist auf ein Bereitstellen von Trainingsausgangsdaten gerichtet, wobei die Trainingsausgangsdaten einen Trainings-Parametersatz aufweisen, welcher Trainings-Parametersatz geeignet ist, mit dem Bildsynthesealgorithmus einen Satz mehrerer verschiedener Visualisierungsbilder basierend auf den Volumendaten zu erzeugen. Ein weiterer Schritt ist auf ein Erzeugen eines Parametersatzes durch Anwenden der trainierten Funktion auf die Volumendaten (und optional eine Patienteninformation und/oder ein Anforderungsprofil und/oder eine Nutzerpräferenz) gerichtet, wobei der Parametersatz geeignet ist, mit dem Bildsynthesealgorithmus einen Satz mehrerer verschiedener Visualisierungsbilder basierend auf den Volumendaten zu erzeugen. Ein weiterer Schritt ist auf ein Vergleichen des Parametersatzes mit dem Trainings-Parametersatz gerichtet. Ein weiterer Schritt ist auf ein Anpassen der trainierten Funktion auf Grundlage des Vergleichs gerichtet.

Gemäß einem weiteren Aspekt wird ein Trainingssystem zum Bereitstellen einer trainierten Funktion bereitgestellt, welches dazu ausgebildet ist, ein oder mehrere Verfahrensschritte des vorgenannten Verfahrens zum Bereitstellen einer trainierten Funktion auszuführen.

Gemäß einem Aspekt wird eine Vorrichtung zum Bereitstellen eines 3D-Modells eines durch Volumendaten repräsentierten dreidimensionalen Objekts bereitgestellt. Die Vorrichtung weist eine Recheneinrichtung und eine Schnittstelle auf. Die Schnittstelle ist dazu ausgebildet, die Volumendaten zu empfangen und das 3D-Modell bereitzustellen. Die Recheneinrichtung ist dazu ausgebildet, einen Bildsynthesealgorithmus zu hosten, welcher Bildsynthesealgorithmus zur Visualisierung des dreidimensionalen Objekts durch Abbilden der Volumendaten auf Visualisierungspixel eines zweidimensionalen Visualisierungsbildes ausgebildet ist. Die Recheneinrichtung ist ferner dazu ausgebildet, einen Parametersatz zur Ansteuerung des Bildsynthesealgorithmus zu ermitteln, welcher Parametersatz geeignet ist, mit dem Bildsynthesealgorithmus einen Satz mehrerer verschiedener Visualisierungsbilder basierend auf dem Volumendatensatz zu erzeugen. Die Recheneinrichtung ist ferner dazu ausgebildet, den Satz mehrerer verschiedener Visualisierungsbilder durch Abbilden der Volumendaten mit dem Bildsynthesealgorithmus unter Verwendung des Parametersatzes zu erzeugen. Die Recheneinrichtung ist ferner dazu ausgebildet, das 3D-Modell basierend auf dem Satz mehrerer Visualisierungsbilder zu berechnen.

Insbesondere kann die Recheneinrichtung der hierin beschriebenen zweiten Recheneinrichtung entsprechen.

Beispielsweise kann die Recheneinrichtung ein Konfigurationsmodul umfassen, das dazu ausgebildet ist, basierend auf den Volumendaten (und optional basierend auf einer Patienteninformation und/oder einem Anforderungsprofil und/oder einer Nutzerpräferenz) den Parametersatz bereitzustellen. Das Konfigurationsmodul kann dazu ausgebildet sein, die trainierte Funktion auszuführen.

Beispielsweise kann die Recheneinrichtung ein Visualisierungsmodul umfassen, das dazu ausgebildet ist, basierend auf dem Parametersatz und den Volumendaten die Visualisierungsbilder zu erzeugen. Das Visualisierungsmodul kann dazu ausgebildet sein, den Bildsynthesealgorithmus auszuführen.

Beispielsweise kann die Recheneinrichtung ein Modellierungsmodul umfassen, das dazu ausgebildet ist, basierend auf den Visualisierungsbildern das 3D-Modell zu erzeugen. Das Modellierungsmodul kann dazu ausgebildet sein, einen Photogrammetriealgorithmus auszuführen.

Die Schnittstelle kann allgemein zum Datenaustausch zwischen der Recheneinrichtung und weiteren Komponenten ausgebildet sein. Die Schnittstelle kann in Form von einer oder mehreren einzelnen Datenschnittstellen implementiert sein, welche ein Hardware- und/oder Software-Interface, z.B. einen PCI-Bus, eine USB-Schnittstelle, eine Fire-Wire-Schnittstelle, eine Zig-Bee- oder eine Bluetooth-Schnittstelle aufweisen können. Die Schnittstelle kann ferner eine Schnittstelle eines Kommunikationsnetzwerks aufweisen, wobei das Kommunikationsnetzwerk ein Local Area Network (LAN), beispielsweise ein Intranet oder ein Wide Area Network (WAN) oder ein Internet aufweisen kann. Entsprechend können die ein oder mehreren Datenschnittstellen eine LAN-Schnittstelle oder eine Wireless LAN-Schnittstelle (WLAN oder Wi-Fi) aufweisen.

Gemäß einem Aspekt weist die Vorrichtung ferner eine Speichereinrichtung auf, die dazu ausgebildet ist, Volumendaten zu speichern und über die Schnittstelle bereitzustellen.

Die Speichereinrichtung kann insbesondere Teil eines sog. Picture Archiving and Communication Systems (PACS) sein. Zusätzlich oder alternativ kann die Speichereinrichtung Teil eines medizinischen Informationssystems, wie eines Krankenhaus- oder Laborinformationssystems sein.

Die Vorteile der vorgeschlagenen Vorrichtung entsprechen im Wesentlichen den Vorteilen des vorgeschlagenen Verfahrens. Merkmale, Vorteile oder alternative Ausführungsformen können ebenso auf die anderen beanspruchten Gegenstände übertragen werden und umgekehrt.

Gemäß einem Aspekt wird ein Computerprogrammprodukt mit einem Computerprogramm bereitgestellt, welches direkt in einen Speicher einer Vorrichtung ladbar ist, mit Programmabschnitten, um alle Schritte des Verfahrens zum Bereitstellen eines 3D-Modells bzw. zum Bereitstellen einer trainierten Funktion nach einem der hierin beschriebenen Aspekte auszuführen, wenn die Programmabschnitte von der Vorrichtung ausgeführt werden.

Gemäß einem Aspekt wird ein computerlesbares Speichermedium bereitgestellt, auf welchem von einer Vorrichtung lesbare und ausführbare Programmabschnitte gespeichert sind, um alle Schritte des Verfahrens zum Bereitstellen eines 3D-Modells bzw. zum Bereitstellen einer trainierten Funktion nach einem der hierin beschriebenen Aspekte auszuführen, wenn die Programmabschnitte von der Vorrichtung ausgeführt werden.

Die Computerprogrammprodukte können dabei eine Software mit einem Quellcode, der noch kompiliert und gebunden oder der nur interpretiert werden muss, oder einen ausführbaren Softwarecode umfassen, der zur Ausführung nur noch in die Verarbeitungseinheit zu laden ist. Durch die Computerprogrammprodukte können die Verfahren schnell, identisch wiederholbar und robust ausgeführt werden. Die Computerprogrammprodukte sind so konfiguriert, dass sie mittels der Recheneinheit die erfindungsgemäßen Verfahrensschritte ausführen können. Die Recheneinheit muss dabei jeweils die Voraussetzungen wie beispielsweise einen entsprechenden Arbeitsspeicher, einen entsprechenden Prozessor, eine entsprechende Grafikkarte oder eine entsprechende Logikeinheit aufweisen, sodass die jeweiligen Verfahrensschritte effizient ausgeführt werden können.

Die Computerprogrammprodukte sind beispielsweise auf einem computerlesbaren Speichermedium gespeichert oder auf einem Netzwerk oder Server hinterlegt, von wo sie in den Prozessor der jeweiligen Recheneinheit geladen werden können, der mit der Recheneinheit direkt verbunden oder als Teil der Recheneinheit ausgebildet sein kann. Weiterhin können Steuerinformationen der Computerprogrammprodukte auf einem computerlesbaren Speichermedium gespeichert sein. Die Steuerinformationen des computerlesbaren Speichermedium können derart ausgebildet sein, dass sie bei Verwendung des Datenträgers in einer Recheneinheit ein erfindungsgemäßes Verfahren durchführen. Beispiele für computerlesbaren Speichermedium sind eine DVD, ein Magnetband oder ein USB-Stick, auf welchem elektronisch lesbare Steuerinformationen, insbesondere Software, gespeichert ist. Wenn diese Steuerinformationen von dem Datenträger gelesen und in eine Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten computerlesbaren Speichermedium ausgehen.

Die Vorteile der vorgeschlagenen Computerprogrammprodukte bzw. der zugehörigen computerlesbaren Medien entsprechen im Wesentlichen den Vorteilen der vorgeschlagenen Verfahren.

Weitere Besonderheiten und Vorteile der Erfindung werden aus den nachfolgenden Erläuterungen von Ausführungsbeispielen anhand von schematischen Zeichnungen ersichtlich. In diesem Zusammenhang genannte Modifikationen können jeweils miteinander kombiniert werden, um neue Ausführungsformen auszubilden. In unterschiedlichen Figuren werden für gleiche Merkmale die gleichen Bezugszeichen verwendet.

Es zeigen:

Fig. 1 eine schematische Darstellung einer ersten Ausführungsform eines Systems zur Bereitstellung eines 3D-Modells,

Fig. 2 ein Ablaufdiagramm eines Verfahrens zur Bereitstellung eines 3D-Modells gemäß einer Ausführungsform,

Fig. 3 ein Datenflussdiagramm eines Verfahrens zur Bereitstellung eines 3D-Modells gemäß einer Ausführungsform,

Fig. 4 eine trainierte Funktion zum Erzeugen eines Parametersatzes zur Eingabe in einen Bildsynthesealgorithmus,

Fig. 5 eine schematische Darstellung einer Ausführungsform eines Systems zum Bereitstellen der trainierten Funktion, und

Fig. 6 ein Ablaufdiagramm eines Verfahrens zur Bereitstellung einer trainierten Funktion zur Erzeugung eines Parametersatzes.

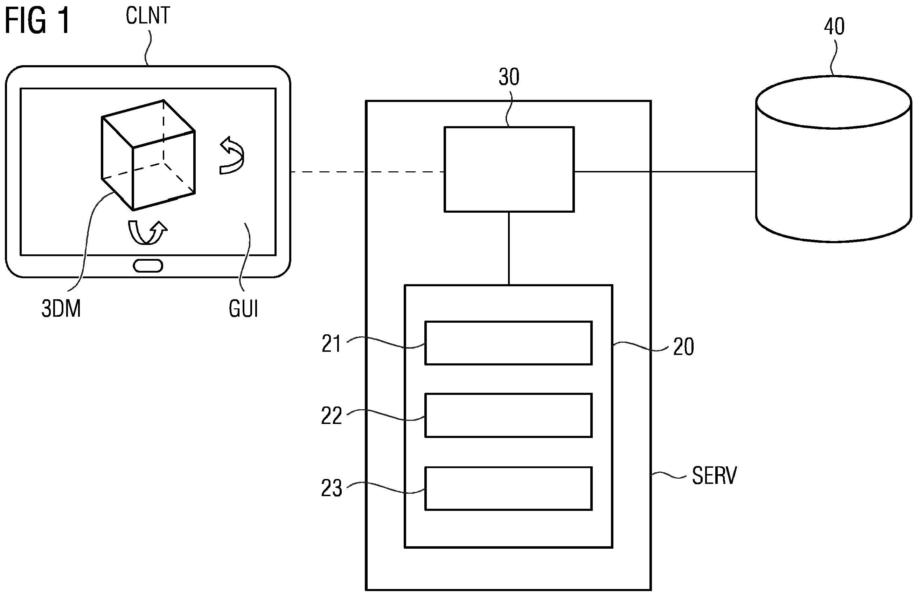

In Figur 1 ist ein System 1 zur Bereitstellung eines 3D-Modells eines Objekts gemäß einer Ausführungsform dargestellt. Das System 1 weist einen Server SERV (als Beispiel einer zweiten Recheneinheit), eine Schnittstelle 30, ein Nutzerendgerät oder Client CLNT (als Beispiel einer ersten Recheneinrichtung) und eine Speichereinrichtung 40 auf. Der Server SERV ist grundsätzlich zur Berechnung eines 3D-Modells eines dreidimensionalen Objekts auf Grundlage das dreidimensionale Objekt beschreibender Volumendaten VD ausgebildet. Die Volumendaten VD können dem Server SERV über die Schnittstelle 30 von der Speichereinrichtung 40 bereitgestellt werden.

Die Speichereinrichtung 40 kann als zentrale oder dezentrale Datenbank ausgebildet sein. Die Speichereinrichtung 40 kann insbesondere Teil eines Serversystems sein. Im medizinischen Umfeld kann die Speichereinrichtung 40 insbesondere Teil eines medizinischen Informationssystems wie etwa eines Krankenhausinformationssystem (ein englischer Fachbegriff hierfür ist hospital information system oder kurz HIS) und/der eines PACS-Systems sein (PACS steht dabei für picture archiving and communication system) und/oder eines Labor-Informationssystem (LIS) sein. Die Speichereinrichtung 40 kann ferner als sog. Cloud-Speicher ausgebildet sein.

In der Speichereinrichtung 40 können die Volumendaten VD des dreidimensionalen Objekts gespeichert sein. Die Volumendaten VD repräsentieren das dreidimensionale Objekt. Die Volumendaten VD weisen einen dreidimensionalen Datensatz aus einer Mehrzahl an Volumenpixeln, den sog. Voxeln auf. In den Volumendaten VD können ortsaufgelöst eine oder mehrere geometrische und/oder physikalische Eigenschaften des dreidimensionalen Objekts codiert sein. Beispielsweise können die Voxelwerte ein Maß für die lokale Dichte des dreidimensionalen Objekts am Ort des Voxels darstellen. Die Volumendaten VD können durch ein entsprechendes Bildgebungsverfahren erzeugt worden sein, beispielsweise durch Röntgendurchleuchtung oder computertomographische Verfahren.

Bei medizinischen Anwendungen können die Volumendaten VD durch ein medizinisches Bildgebungsverfahren erzeugt worden sein. Die Volumendaten VD können dann ein Körperteil eines Patienten repräsentieren und beispielsweise ein oder mehrere Organe des Patienten zeigen. Beispielsweise können die Volumendaten VD durch Röntgendurchleuchtung, Computertomographie (CT), Magnetresonanztomographie (MR), Ultraschall und/oder Positronen-Emissionstomographie (PET) erzeugt worden sein. Die Volumendaten VD können beispielsweise im DICOM-Format formatiert sein. DICOM steht dabei für Digital Imaging and Communications in Medicine und bezeichnet einen offenen Standard zur Speicherung und zum Austausch von Informationen im medizinischen Bilddatenmanagement.

Das Bereitstellen der Volumendaten VD kann beispielsweise ein Laden der Volumendaten VD in einen nicht gezeigten Arbeitsspeicher des Servers SERV umfassen.

Zusätzlich können in dem System 1 weitere Informationen vorhanden sein, die für die Erzeugung des dreidimensionalen Modells 3DM relevant sind. Dies sind zum Beispiel spezifische Anforderungsprofile AP für die Erzeugung des 3D-Modells 3DM, gewisse Nutzerpräferenzen NP hinsichtlich der Erzeugung des 3D-Modells, oder weitere Informationen betreffend das zu modellierende Objekt. Im medizinischen Anwendungsfall kann letzteres beispielsweise eine Patienteninformation PI sein. Zusatzinformationen können in der Speichereinrichtung 40 (oder dem medizinischen Informationssystem) gespeichert sein oder von einem Nutzer des Nutzerendgeräts CLNT bereitgestellt werden.

Der Server SERV ist dazu ausgebildet, basierend auf den Volumendaten VD ein dreidimensionales Modell 3DM des in den Volumendaten VD gezeigten Objekts zu berechnen. Dabei kann der Server SERV dazu ausgebildet sein, die Zusatzinformationen zu berücksichtigen. Der Server SERV kann einen oder mehrere Prozessoren aufweisen. Die Prozessoren können als eine Central Processing Unit (CPU), eine Graphics Processing Unit (GPU), ein digitaler Signalprozessor (DSP), ein Bildverarbeitungsprozessor, ein integrierter (digitaler oder analoger) Schaltkreis oder Kombinationen der vorgenannten Komponenten implementiert sein. Der Server SERV kann als einzelne Komponente implementiert sein oder mehrere Komponenten aufweisen, die parallel oder seriell arbeiten. Alternativ kann der Server SERV eine reale oder virtuelle Gruppe von Rechnern aufweisen, wie etwa ein Cluster oder eine Cloud. Je nach Ausführungsform kann der Server SERV als lokaler Server oder als Cloudserver ausgebildet sein. Der Server SERV ist z.B. durch computerlesbare Instruktionen, durch Design und/oder Hardware derart ausgebildet, dass er ein oder mehrere Verfahrensschritte gemäß Ausführungsformen der Erfindung ausführen kann.

Der Server SERV ist insbesondere dazu ausgebildet, das 3D-Modell 3DM nicht direkt aus den Volumendaten VD zu berechnen, sondern über einen Zwischenschritt. Dieser Zwischenschritt sieht vor, zunächst einen Satz S an zweidimensionalen Visualisierungsbildern VB aus den Volumendaten VD zu erzeugen. Dazu findet bevorzugt ein physikalisches Renderer-Verfahren Anwendung, das auf der Simulation von optischen Strahlengängen beruhen kann. Dadurch können sehr realistische Abbildungen der Volumendaten VD erzeugt werden. Der Server SERV ist ferner dazu ausgebildet, aus diesen Bildern dann das 3D-Modell 3DM zu berechnen. Gegenüber einer direkten Berechnung des 3D-Modells 3DM aus den Volumendaten VD hat das den Vorteil, dass Abbildungseffekte wie Transparenz, Shading, Color-Bleeding, Okklusionseffekte usw. berücksichtigt werden können. Gerade bei medizinischen Bilddaten ist eine sinnvolle Berechnung eines 3D-Modells direkt aus den Volumendaten VD oft nicht ohne weiteres möglich, da verschiedene Gewebe durch medizinische Bildgebungsverfahren nicht trennscharf genug aufgelöst werden und erst durch ein Rendering-Verfahren herausgearbeitet werden müssen.

Das 3D-Modell stellt in gewisser Weise einen gegenüber den Volumendaten VD reduzierten oder komprimierten Datensatz da, der die wesentlichen Eigenschaften für die Betrachtung des Objekts enthält. Dies kann beispielsweise eine Sammlung von Punkten im 3D-Raum sein, die durch verschiedene geometrische Einheiten wie Dreiecke, Linien, gekrümmte Oberflächen usw. verbunden sind und so die für den Betrachter relevanten Flächen charakterisieren. Demgegenüber kann z.B. auf eine innere Struktur des Objekts verzichtet werden.

Zur Bereitstellung des 3D-Modells 3DM basierend auf den Eingangsdaten kann der Server SERV bzw. dessen Recheneinheit 20 verschiedene Module 21, 22, und 23 aufweisen. Die vorgenommene Unterteilung der Recheneinheit 20 in Module 21-23 dient dabei lediglich der einfacheren Erklärung der Funktionsweise der Recheneinheit 20 bzw. des Servers SERV und ist nicht beschränkend zu verstehen. Die Module 21-23 bzw. deren Funktionen können auch in einer Einheit zusammengefasst sein. Die Module 21-23 können dabei insbesondere auch als Computerprogrammprodukte oder Computerprogrammsegmente aufgefasst werden, welche bei Ausführung in der Recheneinheit 20 bzw. dem Server SERV ein oder mehrere der nachstehend beschriebenen Verfahrensschritte realisieren.

Das Modul 21 kann als Konfigurationsmodul 21 aufgefasst werden. Das Konfigurationsmodul 21 kann einen Konfigurationsalgorithmus KA implementieren, hosten oder steuern, der dazu ausgebildet ist, basierend auf den Eingangsdaten, also den Volumendaten VD und ggf. Zusatzinformationen, einen Parametersatz PS zur Steuerung eines Bildsynthesealgorithmus BSA ermittelt. Der Parametersatz PS enthält dabei Angaben, die den Bildsynthesealgorithmus BSA dazu veranlassen, den Satz S Visualisierungsbilder VB auszugeben. Gemäß einigen Ausführungsformen kann der Konfigurationsalgorithmus KA eine trainierte Funktion aufweisen.

Das Modul 22 kann als Visualisierungsmodul 22 oder "Volumenwiedergabe-Engine" aufgefasst werden. Das Visualisierungsmodul 22 kann dabei einen Bildsynthesealgorithmus BSA implementieren, hosten oder steuern, der dazu ausgebildet ist, die Volumendaten VD auf das Visualisierungsbild VB bzw. die Pixel des Visualisierungsbildes VB abzubilden. Zur Berechnung der Abbildung kann der Bildsynthesealgorithmus BSA seinerseits verschiedene Module aufweisen. Beispielsweise kann der Bildsynthesealgorithmus BSA ein Ray-Casting-Modul umfassen, bei dem die Pixel der Visualisierungsbilder VB mit dem Verfahren des Ray-Castings berechnet werden. Ferner kann der Bildsynthesealgorithmus BSA ein Path-Tracing-Modul aufweisen, bei dem die Pixel der Visualisierungsbilder VB nach dem Verfahren des Path-Tracings berechnet werden. Zusätzlich können sich einzelne Module des Bildsynthesealgorithmus BSA auf ergänzende Abbildungseffekte beziehen. Diese ergänzenden Abbildungs- effekte können insbesondere bei Ray-Casting Verfahren beispielsweise Effekte der Umgebungsverdeckung, Schatteneffekte, Transluzenzeffekte, Color Bleeding Effekte, Oberflächenschattierungen, komplexe Kameraeffekte und/oder Beleuchtungseffekte durch beliebige Umgebungslichtverhältnisse umfassen.

Das Modul 23 kann als Modellierungsmodul 23 aufgefasst werden. Das Modellierungsmodul 23 ist dazu ausgebildet, aus durch den Bildsynthesealgorithmus BSA berechneten Visualisierungsbildern VB ein 3D-Modell zu berechnen. Das Modellierungsmodul 23 kann dabei einen Modellierungsalgorithmus MA implementieren, hosten oder steuern, der dazu ausgebildet ist, aus den Visualisierungsbilder VB das 3D-Modell 3DM zu berechnen. Insbesondere kann der Modellierungsalgorithmus MA ein Photogrammetrieverfahren implementieren.

Ist das 3D-Modell 3DM berechnet, soll es dem Nutzer angezeigt werden. Dazu wird das 3D-Modell 3DM dem Nutzerendgerät CLNT über die Schnittstelle 30 bereitgestellt. Das Nutzerendgerät CLNT kann einen Ausgabeeinrichtung wie einen Bildschirm aufweisen, der zur Anzeige einer grafischen Nutzerschnittstelle GUI zur Darstellung verschiedener Ansichten des 3D-Modells ausgelegt ist. Zusätzlich kann das Nutzerendgerät CLNT eine Eingabeeinrichtung wie einen Touchscreen aufweisen, mit welcher der Nutzer verschiedene Ansichten des 3D-Modells anwählen kann. Das Nutzerendgerät CLNT kann eine Recheneinheit, wie etwa einen Prozessor aufweisen, der dazu ausgebildet ist, basierend auf den ausgewählten Ansichten und dem 3D-Modell 3DM zweidimensionale Visualisierungen zu berechnen. In beispielhaften Ausführungsformen ist das Nutzerendgerät CLNT ein Smartphone oder ein Tablet PC oder ein Laptop oder ein Desktop PC.

Die Schnittstelle 30 kann ein oder mehrere einzelne Datenschnittstellen aufweisen, die den Datenaustausch zwischen den Komponenten SERV, CLNT, 40 des Systems 1 gewährleisten. Die ein oder mehreren Datenschnittstellen können ein Hardware-und/oder Software-Interface, z.B. einen PCI-Bus, eine USB-Schnittstelle, eine Fire-Wire-Schnittstelle, eine ZigBee-oder eine Bluetooth-Schnittstelle aufweisen. Die ein oder mehreren Datenschnittstellen können eine Schnittstelle eines Kommunikationsnetzwerks aufweisen, wobei das Kommunikationsnetzwerk ein Local Area Network (LAN), beispielsweise ein Intranet oder ein Wide Area Network (WAN) oder ein Internet aufweisen kann. Entsprechend können die ein oder mehreren Datenschnittstellen eine LAN-Schnittstelle oder eine Wireless LAN-Schnittstelle (WLAN oder Wi-Fi) aufweisen. Insbesondere ist die Schnittstelle zwischen Nutzerendgerät CLNT und Server SERV eine Internet-Schnittstelle und die Datenübertragung zwischen Nutzerendgerät CLNT und Server SERV erfolgt über das Internet.

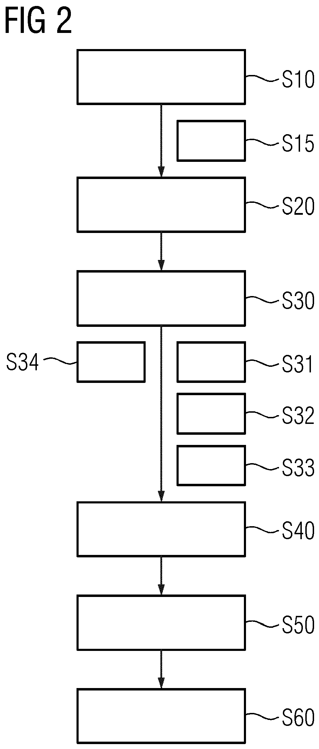

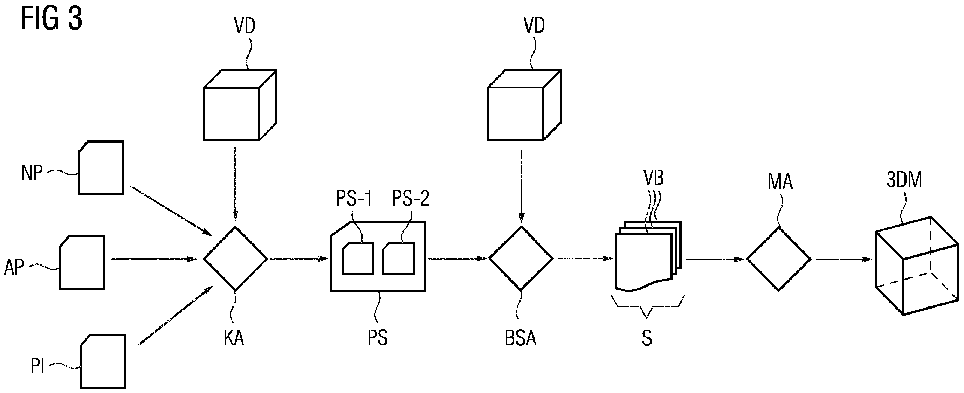

In Figur 2 ist ein schematisches Ablaufdiagramm eines Verfahrens zur Visualisierung eines dreidimensionalen Objekts dargestellt. Die Reihenfolge der Verfahrensschritte ist weder durch die dargestellte Abfolge noch durch die gewählte Nummerierung beschränkt. So kann die Reihenfolge der Schritte ggf. vertauscht und einzelne Schritte können weggelassen werden. In Figur 3 ist ein zugehöriges Datenflussdiagramm schematisch dargestellt. In Figuren 2 und 3 ist das Verfahren exemplarisch anhand von medizinischen Bilddaten als Volumendaten VD dargestellt. Es versteht sich, dass das Verfahren weitgehend auch auf beliebige andere Arten von Bilddaten anwendbar ist.

Ein erster Schritt S10 ist auf das Bereitstellen der Volumendaten VD gerichtet. Die Bereitstellung kann dabei durch einen Abruf der Volumendaten VD von der Speichereinrichtung 40 und/oder einem Laden der Volumendaten VD in den Server SERV realisiert sein. Die Volumendaten VD stellen dabei ein dreidimensionales Objekt dar. Im diskutierten Beispiel handelt es sich dabei um ein Körperteil eines Patienten.

In einem optionalen Teilschritt S15 können Zusatzinformationen zur zielgerichteten Erzeugung des Satzes S der Visualisierungsbilder VB und damit zur zielgerichteten Berechnung des 3D-Modells 3DM bereitgestellt werden. Die Zusatzinformationen können beispielsweise eine Patienteninformation PI, ein Anforderungsprofil AP und/oder eine Nutzerpräferenz NP umfassen.

Die Patienteninformation PI kann beispielsweise einen medizinischen Befundsbericht des Patienten, und/oder eine elektronische Patientenakte des Patienten und/oder eine Befundungsaufgabe zur Erstellung eines Befundes für den Patienten durch einen Nutzer umfassen. Damit kann die Patienteninformation PI Angaben enthalten, die für die Visualisierung und Modellerzeugung relevant sind, wie etwa eine Angabe eines zu befundenden Körperteils, eine oder mehrere Verdachtsdiagnosen betreffend ein oder mehrere Körperteile, eine Angabe eines medizinischen Befunds usw. Insbesondere kann die Patienteninformation PI ein oder mehrere strukturierte oder unstrukturierte Textdokumente umfassen, die natürliche Sprache enthalten. Solche Informationen können von der Speichereinrichtung 40 bereitgestellt bzw. heruntergeladen werden.

Anforderungsprofile AP können allgemein Angaben darüber umfassen, welche verschiedenen Einzelbilder zur Erzeugung eines bestimmten 3D-Modells 3DM erforderlich sind. Dies kann eine Anzahl der Visualisierungsbilder VB oder eine Angabe der abzubildenden Ansichten umfassen. Dabei können verschiedene Arten von Modellen 3DM jeweils verschiedene Anforderungsprofile AP aufweisen. Bei der Auswahl eines Modells 3DM kann folglich das zugehörige Anforderungsprofil AP ausgewählt und bereitgestellt werden. Anforderungsprofile AP können im Server SERV gespeichert sein oder von der Speichereinrichtung 40 bereitgestellt bzw. heruntergeladen werden.

Nutzerpräferenzen NP können allgemein bevorzugte Nutzereinstellungen für die Berechnung des 3D-Modells 3DM umfassen, wie etwa Abbildungsparameter (Szenenillumination, Auflösung, verfügbare Ansichten, Transferfunktionen, etc.) oder die Art des vom Nutzer gewünschten 3D-Modells 3DM. Nutzerpräferenzen NP können beispielsweise von dem Nutzerendgerät CLNT bereitgestellt werden. Alternativ können Nutzerpräferenzen NP im Server SERV gespeichert sein oder von der Speichereinrichtung 40 bereitgestellt bzw. heruntergeladen werden.

Ein weiterer Schritt S20 ist auf das Bereitstellen des Bildsynthesealgorithmus BSA gerichtet. Die Bereitstellung kann dabei durch Vorhalten und/oder Aufruf des Bildsynthesealgorithmus BSA in bzw. von einem beliebigen Speicher (z.B. einer Speichereinrichtung 40 eines medizinischen Informationssystems oder einem beliebigen Speicher des Servers SERV) und/oder einem Laden des Bildsynthesealgorithmus BSA in die Recheneinheit 20 des Servers SERV realisiert sein.

In einem weiteren Schritt S30 wird basierend auf den verfügbaren Eingabedaten, also den Volumendaten VD sowie optional der Patienteninformation PI, der Nutzerpräferenz NP und/oder dem Anforderungsprofil AP ein Parametersatz PS ermittelt. Der Parametersatz PS ist geeignet, mit dem Bildsynthesealgorithmus BSA den Satz S an Visualisierungsbildern VB zu erzeugen, aus dem ein 3D-Modell 3DM berechnet werden kann.

Der Parametersatz PS kann demzufolge als Satz an Steuerbefehlen aufgefasst werden, mit dem der Bildsynthesealgorithmus BSA zur Erzeugung des Satzes S an Visualisierungsbildern VB angesteuert werden kann. Im Parametersatz PS können folglich entsprechende Einstellungen für den Bildsynthesealgorithmus BSA codiert sein. Gemäß beispielhaften Ausführungsformen weist der Parametersatz PS zwei unterschiedliche Arten Parameter auf. Einerseits sind dies "globale" Parameter, die für alle Visualisierungsbilder VB eines Satzes S gelten. Dies betrifft z.B. die Szenenillumination, die Transferfunktion, den Kontrast, die Schärfe, eine Transparenz ein oder mehrerer Bereiche der Volumendaten VD, eine Schnittebene durch die Volumendaten VD, eine oder mehrere Zusatzinformationen zur Einblendung in die Visualisierungsbilder VB usw. Diese "globalen" Parameter sind in der ersten Parametrierung PS-1 zusammengefasst. Anderseits umfasst der Parametersatz PS auch eine zweite Parametrierung PS-2, in welcher Anweisungen zum Erzeugen der verschiedenen Ansichten bzw. Perspektiven für das anschließende Berechnen des 3D-Modells 3DM codiert sind.

Optional kann in einem Teilschritt S31 das zu berechnende 3D-Modell 3DM festgelegt werden. Dies kann automatisch basierend auf den Volumendaten VD bzw. der optionalen Patienteninformation PI und/oder dem optionalen Anforderungsprofil AP erfolgen. So kann z.B. festgelegt sein, dass für eine bestimmte Patienteninformation PI (z.B. einen bestimmten zu erstellenden Befund) ein bestimmtes 3D-Modell zu erzeugen ist. Das zu erzeugende 3D-Modell kann dabei in Schritt S31 automatisch aus einer Mehrzahl verschiedener vorbestimmter 3D-Modelle ausgewählt werden. Alternativ kann eine Auswahl eines bestimmten 3D-Modells 3DM durch einen Nutzer des Nutzerendgeräts CLNT erfolgen und z.B. über die Nutzerpräferenz NP an den Server SERV weitergegeben werden.

Optional kann in Schritt S30 eine automatische Segmentierung der Volumendaten VD für die Visualisierung und damit das 3D-Modell 3DM erfolgen. Dazu können in einem optionalen Teilschritt S32 zunächst eines oder mehrere Auswahl-Organe zur Darstellung in dem 3D-Modell 3DM identifiziert werden. Dies kann beispielsweise basierend auf der Patienteninformation PI erfolgen. Findet in der Patienteninformation PI z.B. die Lunge Erwähnung, kann daraus auf die Lunge als Auswahl-Organ geschlossen werden. Hierfür kann die Patienteninformation PI beispielsweise mit einem Computerlinguistikalgorithmus analysiert werden, der dazu ausgebildet ist, ein oder mehrere Schlagworte oder Begriffe in der Patienteninformation PI zu erkennen. In einem weiteren Teilschritt S33 können eine oder mehrere Segmentierungsmasken zur Anwendung auf die Volumendaten VD ermittelt werden. Die Segmentierungsmasken sind dazu ausgebildet, die zu dem Auswahl-Organ gehörenden Bilddaten in den Volumendaten VD zu segmentieren, d.h. zu identifizieren.

Der Parametersatz PS kann wie erwähnt durch einen entsprechend angepassten Konfigurationsalgorithmus KA basierend auf den Eingabedaten (Volumendaten VD bzw. optional Patienteninformation PI, Nutzerpräferenz NP und/oder Anforderungsprofil AP) ermittelt werden. Der Konfigurationsalgorithmus KA kann einen Computerlingustikalgorithmus umfassen, der zur algorithmischen Verarbeitung von natürlicher Sprache in Form von Text- oder Sprachdaten ausgebildet ist. Ferner kann der Konfigurationsalgorithmus KA eine trainierte Funktion TF umfassen, die dazu ausgebildet ist, basierend auf den Eingangsdaten den Parametersatz PS zu erzeugen. In einem optionalen Schritt S34 kann eine solche trainierte Funktion entsprechend bereitgestellt werden.

In Schritt S40 wird basierend auf dem Parametersatz PS und den Volumendaten VD der Satz S Visualisierungsbilder VB erzeugt. Dazu werden die Volumendaten VD und der Parametersatz PS in den Bildsynthesealgorithmus BSA eingegeben. Optional kann dabei eine in Schritt S30 ermittelte Segmentierungsmaske berücksichtigt werden.

In Schritt S50 werden die in Schritt S40 erzeugten Visualisierungsbilder VD dazu verwendet, ein 3D-Modell 3DM des in den Volumendaten VD gezeigten Objekts zu berechnen. Dazu werden die Visualisierungsbilder VB in einen Modellierungsalgorithmus MA eingegeben, der zur Berechnung eines 3D-Modells eines Objekts basierend auf verschiedenen zweidimensionalen Ansichten ausgebildet ist. Mit anderen Worten wandelt der Modellierungsalgorithmus MA mehrere zweidimensionale Visualisierungsbilder VB in das dreidimensionale Modell 3DM um.

Insbesondere kann der Modellierungsalgorithmus MA ein Verfahren der Photogrammetrie implementieren. Der Modellierungsalgorithmus MA ist dazu ausgebildet, aus dem Satz S an Visualisierungsbildern VB durch Bildmessung die Lage und Form des dreidimensionalen Objekts zu bestimmen. Dazu kann die Abbildungsgeometrie zum Zeitpunkt der Berechnung der Visualisierungsbilder VB wiederhergestellt werden. Diese Wiederherstellung kann z.B. nach den Gesetzen der Zentralprojektion unter Einhaltung der Komplanarität erfolgen. Dabei legt jedes Bild für einen abgebildeten Punkt zusammen mit dem Projektionszentrum der jeweiligen virtuellen Kamera eine Richtung zum Objektpunkt fest. Bei bekannter Orientierung der virtuellen Kamera und bekannter Abbildungsgeometrie kann dann jeder Strahl im Raum beschrieben werden. Durch Verwendung von mindestens zwei homologen (korrespondierenden) Bildpunkten zweier verschiedener Aufnahmepositionen kann der Modellierungsalgorithmus MA bei Kenntnis der gegenseitigen Lage (relative Orientierung) die beiden Strahlen zum Schnitt bringen und so jeden Objektpunkt dreidimensional berechnen.

In Schritt S60 wird das 3D-Modell 3DM schließlich bereitgestellt. Dazu kann es an das Nutzerendgerät CLNT übermittelt oder zum Download durch das Nutzerendgerät CLNT bereitgestellt werden. Zum Download bereitgestellt werden kann das 3D-Modell 3DM beispielsweise von einer entsprechenden Speichereinrichtung 40 des medizinischen Informationssystems, wie etwa einem Cloud-Speicher.

Ferner kann das 3D-Modell 3DM mit einer Viewing-Applikation bereitgestellt werden. Die Viewing-Applikation kann eine graphische Nutzerschnittstelle GUI bereitstellen. Über die grafische Nutzerschnittstelle GUI kann der Nutzer beispielsweise verschiedene Ansichten des 3D-Modells 3DM auswählen und sich anzeigen lassen. Die Ansichten stellen also (ähnlich wie die Visualisierungsbilder VB) zweidimensionale "Renderings" von dreidimensionalen Ausgangsdaten dar.

Die Viewing-Applikation kann beispielsweise am Nutzerendgerät CLNT aktiviert werden, indem der Nutzer sie von einem App-Store herunterlädt und/oder lokal ausführt. Alternativ kann die Viewing-Applikation als Web-Applikation ausgebildet sein, die von dem Server und/oder dem medizinischen Informationssystem gehostet wird.

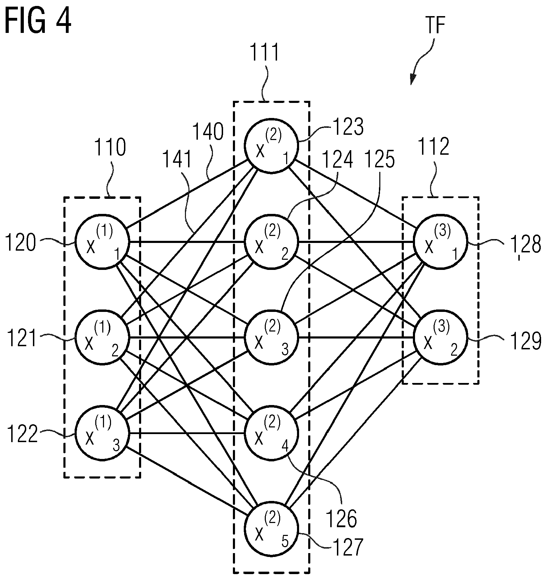

Figur 4 zeigt eine beispielhafte Darstellung einer trainierten Funktion TF, wie sie in Schritt S30 zur Ermittlung eines Parametersatzes PS eingesetzt werden kann. Die trainierte Funktion TF erhält die Volumendaten VB bzw. optional die Patienteninformation PI, die Nutzerpräferenz NP und/oder das Anforderungsprofil AP als Eingabe und gibt als Ausgabe den Parametersatz PS also mit anderen Worten Steuerbefehle zur Ansteuerung des Visualisierungsmoduls 22 bzw. Bildsynthesealgorithmus BSA aus.

In dem gezeigten Ausführungsbeispiel ist die trainierte Funktion TF als neuronales Netz ausgebildet. Das neuronale Netz kann auch als künstliches neuronales Netz, künstliches neuronales Netzwerk oder neuronales Netzwerk bezeichnet werden.

Das neuronale Netz 100 umfasst Knoten 120,..., 129 und Kanten 140,141, wobei jede Kante 140,141 eine gerichtete Verbindung von einem ersten Knoten 120,...,129 zu einem zweiten Knoten 120,..., 129 ist. Im Allgemeinen sind der erste Knoten 120,...,129 und der zweite Knoten 120,...,129 unterschiedliche Knoten, es ist auch möglich, dass der erste Knoten 120,...,129 und der zweite Knoten 120,...,129 identisch sind. Eine Kante 140,141 von einem ersten Knoten 120,...,129 zu einem zweiten Knoten 120,...,129 kann auch als eingehende Kante für den zweiten Knoten und als ausgehende Kante für den ersten Knoten 120,...,129 bezeichnet werden.

Das neuronale Netz 100 antwortet auf Eingabewerte $x^{(1)}_1$, $x^{(1)}_2$, $x^{(1)}_3$ zu einer Vielzahl von Eingangsknoten 120, 121, 122 der Eingangsschicht 110. Die Eingabewerte $x^{(1)}_1$, $x^{(1)}_2$, $x^{(1)}_3$ werden angewendet, um eine oder eine Vielzahl von Ausgaben $x^{(3)}_1$, $x^{(3)}_2$ zu erzeugen. Der Knoten 120 ist beispielsweise über eine Kante 140 mit dem Knoten 123 verbunden. Der Knoten 121 ist beispielsweise über die Kante 141 mit dem Knoten 123 verbunden.

Das neuronale Netz 100 lernt in diesem Ausführungsbeispiel, indem es die Gewichtungsfaktoren $w_{i,j}$ (weights) der einzelnen Knoten basierend auf Trainingsdaten anpasst. Mögliche Eingabewerte $x^{(1)}_1$, $x^{(1)}_2$, $x^{(1)}_3$ der Eingangsknoten 120, 121, 122 können beispielsweise die einzelnen Feldgrößen $\tilde{E}_{BC}$, $\tilde{H}_{BC}$, $\tilde{E}_i$, $\tilde{H}_i$ sein und/oder eine Untersuchungsinformation UI (sofern vorhanden).

Das neuronale Netz 100 gewichtet die Eingabewerte der Eingangsschicht 110 basierend auf dem Lernprozess. Die Ausgabewerte der Ausgangsschicht 112 des neuronalen Netzes 100 entsprechen bevorzugt einer Feldinformation FI, auf deren Grundlage sich das der Signatur S zugrunde liegende elektrische und/oder magnetische Feld zumindest teilweise unterdrücken lässt. Die Ausgabe kann über einen einzelnen oder eine Vielzahl von Ausgabeknoten $x^{(3)}_1$, $x^{(3)}_2$ in der Ausgabeschicht 112 erfolgen.

Das künstliche neuronale Netz 100 umfasst bevorzugt eine versteckte Schicht 111, die eine Vielzahl von Knoten $x^{(2)}_1$, $x^{(2)}_2$, $x^{(2)}_3$ umfasst. Es können mehrere versteckte Schichten vorgesehen sein, wobei eine versteckte Schicht Ausgabewerte einer anderen versteckten Schicht als Eingabewerte verwendet. Die Knoten einer versteckten Schicht 111 verrichten mathematische Operationen. Ein Ausgabewert eines Knotens $x^{(2)}_1$, $x^{(2)}_2$, $x^{(2)}_3$ entspricht dabei einer nicht-linearen Funktion f seiner Eingabewerte $x^{(1)}_1$, $x^{(1)}_2$, $x^{(1)}_3$ und der Gewichtungsfaktoren $w_{i,j}$. Nach dem Erhalt von Eingabewerten $x^{(1)}_1$, $x^{(1)}_2$, $x^{(1)}_3$ führt ein Knoten $x^{(2)}_1$, $x^{(2)}_2$, $x^{(2)}_3$ eine Summierung einer mit den Gewichtungsfaktoren $w_{i,j}$ gewichteten Multiplikation jedes Eingabewerts $x^{(1)}_1$, $x^{(1)}_2$, $x^{(1)}_3$ durch, wie durch folgende Funktion bestimmt:

$$x_j^{(n+1)} \;=\; f\!\left( \sum_i x_i^{(n)} \cdot w_{i,j}^{(m,n)} \right).$$

Der Wichtungsfaktor $w_{i,j}$ kann insbesondere eine reelle Zahl, insbesondere im Intervall von [-1;1] oder [0;1] liegen. Der Wichtungsfaktor $w_{i,j}^{(m,n)}$ bezeichnet das Gewicht der Kante zwischen dem i-ten Knoten einer m-ten Schicht 110,11,112 und einem j-ten Knoten der n-ten Schicht 110,111,112.

Insbesondere wird ein Ausgabewert eines Knotens $x^{(2)}_1$, $x^{(2)}_2$, $x^{(2)}_3$ als Funktion f einer Knoten-Aktivierung, beispielsweise eine Sigmoidalfunktion oder eine lineare Rampenfunktion gebildet. Die Ausgabewerte $x^{(2)}_1$, $x^{(2)}_2$, $x^{(2)}_3$ werden an den bzw. die Ausgabeknoten 128,129 übertragen. Erneut wird eine Summierung einer gewichteten Multiplikation jedes Ausgabewertes $x^{(2)}_1$, $x^{(2)}_2$, $x^{(2)}_3$ als Funktion der Knoten-Aktivierung f und damit die Ausgabewerte $x^{(3)}_1$, $x^{(3)}_2$ berechnet.

Das hier gezeigte neuronale Netz TF ist ein Feedforward neuronales Netz, bei dem alle Knoten 111 die Ausgabewerte einer vorherigen Schicht in Form ihrer gewichteten Summe als Eingabewerte verarbeiten.

Selbstredend können erfindungsgemäß auch andere neuronale Netztypen zum Einsatz kommen, bspw. Feedback-Netze, bei denen ein Eingabewert eines Knotens gleichzeitig auch sein Ausgabewert sein kann.

Das neuronale Netz TF kann mittels einer Methode des überwachten Lernens trainiert werden, um die Feldinformation FI bereitzustellen. Eine bekannte Vorgehensweise ist die Back-Propagation, die für alle Ausführungsbeispiele der Erfindung angewandt werden kann. Während des Trainings wird das neuronale Netz TF auf Trainingseingabedaten bzw. -werten angewandt und muss entsprechende, vorher bekannte Trainingsausgabedaten bzw. -werte erzeugen. Iterativ werden mittlere quadratische Fehler (mean square error - "MSE") zwischen berechneten und erwarteten Ausgabewerten berechnet und einzelne Gewichtungsfaktoren so lange angepasst, bis die Abweichung zwischen berechneten und erwarteten Ausgabewerten unterhalb einer vorbestimmten Schwelle liegt.

Zur Bereitstellung von Trainingsdaten kann beispielsweise auf Parametersätze PS zurückgegriffen werden, die von Visualisierungsexperten für einen bestimmten medizinischen Kontext und für bestimmte Volumendaten VD erstellt wurden.

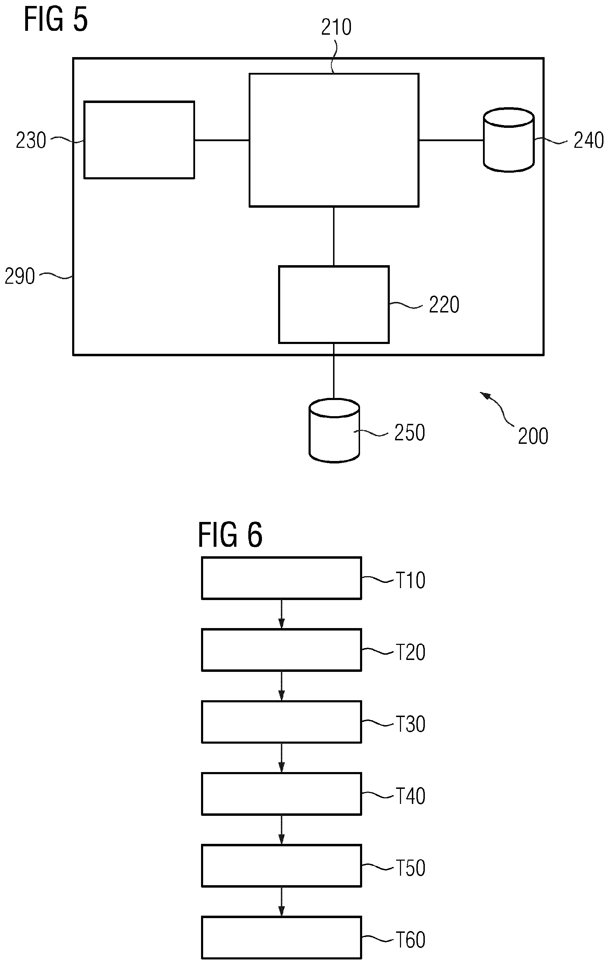

Figur 5 zeigt eine Ausführungsform eines Systems 200 zum Trainieren bzw. Bereitstellen der trainierten Funktion TF. Das System 200 umfasst einen Prozessor 210, eine Schnittstelle 220, einen Arbeitsspeicher 230, eine Speichereinrichtung 240 und eine Datenbank 250. Der Prozessor 210, die Schnittstelle 220, der Arbeitsspeicher 230 und die Speichereinrichtung 240 können als ein Computer 290 ausgebildet sein. Der Prozessor 210 steuert den Betrieb des Computers 290 beim Trainieren der trainierten Funktion TF. Insbesondere kann der Prozessor 210 derart ausgebildet sein, dass er die in Figur 6 dargestellten Verfahrensschritte ausführt. Die Instruktionen können im Arbeitsspeicher 230 oder in der Speichereinrichtung 240 gespeichert und/oder in den Arbeitsspeicher 230 geladen werden, wenn die Ausführung der Instruktionen gewünscht wird. Die Speichereinrichtung 240 kann als ein lokaler Speicher oder ein entfernter Speicher ausgebildet sein, auf den über ein Netzwerk zugegriffen werden kann. Die in Figur 6 dargestellten Verfahrensschritte können durch Computerprogrammprodukte definiert werden, die in dem Arbeitsspeicher 230 und/oder der Speichereinrichtung 240 gespeichert sind.

Die Datenbank 250 kann als Cloud-Speicher oder lokaler Speicher implementiert sein, die mit dem Computer 290 über die drahtlose oder drahtgebundene Schnittstelle 220 in Verbindung steht. Die Datenbank 250 kann insbesondere auch Teil des Computers 290 sein. Die Datenbank 250 dient als Archiv für die (Trainings-)Volumendaten, Trainings-Zusatzinformationen (Patienteninformation PI, Nutzerpräferenz NP und/oder Anforderungsprofil AP) und/oder zugehörige Trainings-Parametersätze. Ferner kann die Datenbank 250 als Archiv für ein oder mehrere trainierte Funktionen TF dienen.

In Figur 6 ist ein schematisches Ablaufdiagramm eines Verfahrens zur Bereitstellung einer trainierten Funktion TF zur Bereitstellung eines Parametersatzes PS dargestellt. Die Reihenfolge der Verfahrensschritte ist weder durch die dargestellte Abfolge noch durch die gewählte Nummerierung beschränkt. So kann die Reihenfolge der Schritte ggf. vertauscht und einzelne Schritte können weggelassen werden.

Ein erster Schritt T10 ist auf das Bereitstellen einer trainierten Funktion TF gerichtet. Dabei kann die trainierte Funktion TF dem Prozessor 210 über die Schnittstelle 220 von der Datenbank 250 bereitgestellt werden. Die trainierte Funktion TF kann dabei bereits vortrainiert sein, d.h. es wurden bereits ein oder mehrere Parameter der trainierten Funktion TF durch das beschriebene Trainingsverfahren und/oder ein anderes Trainingsverfahren angepasst. Alternativ können der eine oder die mehreren Parameter der trainierten Funktion noch nicht mittels Trainingsdaten angepasst sein, insbesondere können der eine oder die mehreren Parameter durch einen konstanten Wert und/oder durch einen zufälligen Wert vorbelegt sein. Insbesondere können alle Parameter der trainierten Funktion TF noch nicht mittels Trainingsdaten angepasst sein, insbesondere können alle Parameter durch einen konstanten Wert und/oder durch einen zufälligen Wert vorbelegt sein.

Ein zweiter Schritt T20 ist auf das Bereitstellen von Trainingseingangsdaten gerichtet. Da die trainierte Funktion TF im Einsatz einen Parametersatz PS passend zu den jeweiligen Volumendaten VD sowie optional basierend auf der zugehörigen Patienteninformation PI, der zugehörigen Nutzerpräferenz NP und/oder dem zugehörigen Anforderungsprofil AP bereitstellen soll, sind geeignete Trainingseingangsdaten eben Trainings-Volumendaten und solche optionalen Zusatzinformationen.

Schritt T30 ist auf das Bereitstellen von Trainingsausgangsdaten gerichtet. Die Trainingsausgangsdaten sind dabei Trainings-Parametersätze PS. Ein Trainings- Parametersatz PS stellt dabei den für einen Satz Trainings-Volumendaten VD sinnvollen Parametersatz PS dar, der zu einem für die Erstellung eines 3D-Modells 3DM geeigneten Satz S an Visualisierungsbildern VB führt. Die Trainings-Parametersätze PS können z.B. durch einen Experten bereitgestellt werden.

In einem nächsten Schritt T40 werden die Trainingseingangsdaten, also Trainings-Volumendaten VD und ggf. die Zusatzinformationen PI, NP, AP in die trainierte Funktion TF eingegeben. Die trainierte Funktion TF berechnet auf dieser Grundlage einen Parametersatz PS zur Ansteuerung eines Bildsynthesealgorithmus BSA.

In einem nächsten Schritt T50 wird der so berechnete Parametersatz PS mit dem zugehörigen Trainings-Parametersatz PS verglichen. Auf Grundlage dieses Vergleichs dann die trainierte Funktion TF in Schritt T60 angepasst werden. Dies kann beispielsweise auf Grundlage eines Kostenfunktionals geschehen, das Abweichungen des berechneten Parametersatzes PS von dem zugehörigen Trainings-Parametersatz PS bestraft. Ein oder mehrere Parameter der trainierten Funktion TF können dann insbesondere so angepasst werden, dass das Kostenfunktional minimiert wird, beispielsweise mittels einer Rückpropagation (ein englischer Fachbegriff ist "back propagation"). Das Kostenfunktional kann in einer Ausführungsform auf einer paarweisen Differenz von Steuer- oder Visualisierungsparametern des berechneten Parametersatzes PS und dem zugehörigen Trainings-Parametersatz PS beruhen, beispielsweise auf der Summe der quadratischen Abweichungen. Zur Minimierung des Kostenfunktionals wird der Vergleich für verschiedene paarweise Sätze aus berechnetem Parametersatzes PS und zugehörigem Trainings-Parametersatzes PS durchgeführt, bis ein lokales Minimum des Kostenfunktionals erreicht ist und die trainierte Funktion TF zufriedenstellend arbeitet.

Wo noch nicht explizit geschehen, jedoch sinnvoll und im Sinne der Erfindung, können einzelne Ausführungsbeispiele, einzelne ihrer Teilaspekte oder Merkmale mit einander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

**Patentansprüche**

1. Computer-implementiertes Verfahren zum Bereitstellen eines 3D-Modells (3DM) eines durch Volumendaten (VD) repräsentierten dreidimensionalen Objekts mit den folgenden Schritten:

   - Bereitstellen (S10) der Volumendaten (VD),
   - Bereitstellen (S20) eines Bildsynthesealgorithmus (BSA), welcher Bildsynthesealgorithmus (BSA) zur Visualisierung des dreidimensionalen Objekts durch Abbilden der Volumendaten (VD) auf Visualisierungspixel (VP) eines zweidimensionalen Visualisierungsbildes (VB) ausgebildet ist,
   - Ermitteln (S30) eines Parametersatzes (PS) zur Ansteuerung des Bildsynthesealgorithmus (BSA), welcher Parametersatz (PS) geeignet ist, mit dem Bildsynthesealgorithmus (BSA) einen Satz (S) mehrerer verschiedener Visualisierungsbilder (VB) basierend auf dem Volumendatensatz (VD) zu erzeugen,
   - Erzeugen (S40) des Satzes (S) mehrerer verschiedener Visualisierungsbilder (VB) durch Abbilden der Volumendaten (VB) mit dem Bildsynthesealgorithmus (BSA) unter Verwendung des Parametersatzes (PS),
   - Berechnen (S50) des 3D-Modells (3DM) basierend auf dem Satz (S) mehrerer Visualisierungsbilder (VB), und
- Bereitstellen (S60) des 3D-Modells (3DM).

2. Verfahren nach Anspruch 1, bei dem

- der Schritt des Berechnens (S50) des 3D-Modells (3DM) durch Photogrammetrie erfolgt.

3. Verfahren nach einem der vorhergehenden Ansprüche, bei dem

- der Parametersatz (PS) aufweist:

- eine erste Parametrierung (PS-1) der Abbildungs- eigenschaften der Volumendaten (VD), und
- eine zweite Parametrierung (PS-2) zur Erzeugung mehrerer verschiedener Ansichten des dreidimensionalen Objekts mit der gleichen ersten Parametrierung (PS-1), um so die mehreren verschiedenen Visualisierungsbilder (VB) des Satzes (S) zu erzeugen.

4. Verfahren nach Anspruch 3, bei dem

- die erste Parametrierung (PS-2) eine Transferfunktion zur Abbildung der Volumendaten (VD) auf das Visualisierungsbild (VB) und/oder eine Segmentierung des dreidimensionalen Objekts und/oder eine Clippingmaske umfasst.

5. Verfahren nach einem der vorhergehenden Ansprüche, bei dem

- die Schritte des Bereitstellens (S10) der Volumendaten (VD), des Bereitstellens (S20) des Bildsynthesealgorithmus (BSA), des Ermittelns (S30) des Parametersatzes (PS), des Erzeugens (S40) des Satzes (S) und des Berechnens (S50) des 3D-Modells (3DM) in einer ersten Recheneinrichtung (SERV) erfolgen, und
- im Schritt des Bereitstellens (S60) das 3D-Modell (3DM) einer zweiten Recheneinrichtung (CLNT) bereitgestellt wird, welche zweite Recheneinrichtung (CLNT) von der ersten Recheneinrichtung (SERV) verschieden ist, wobei die zweite Recheneinrichtung (CLNT) insbesondere ein tragbares Nutzerendgerät (SP) umfasst.

6. Verfahren nach einem der vorhergehenden Ansprüche, bei dem

- der Bildsynthesealgorithmus (BSA) ein pathtracing-oder ray-casting-Verfahren implementiert.

7. Verfahren nach einem der vorhergehenden Ansprüche, bei dem

- die Volumendaten (VD) durch ein medizinisches Bildgebungsverfahren erzeugt sind und ein oder mehrere Organe eines Patienten repräsentieren, und das Verfahren ferner umfasst:
- Bereitstellen (S15) einer dem Patienten zugeordneten Patienteninformation (PI), wobei
- der Parametersatz (PS) zusätzlich basierend auf der Patienteninformation (PI) ermittelt wird.

8. Verfahren nach Anspruch 7, bei dem

- der Schritt des Bereitstellens (S60) des 3D-Modells ein Bereitstellen des 3D-Modells (3DM) als weitere Patienteninformation (PI), insbesondere in Form eines medizinischen Befundsberichts, für den Patienten umfasst.

9. Verfahren nach Anspruch 7 oder 8, ferner mit den Schritten:

- Bestimmen (S32) eines oder mehrerer Auswahl-Organe zur Darstellung in dem 3D-Modell (3DM) basierend auf der Patienteninformation (PI),
- Erzeugen (S33) einer Segmentierungsmaske zur Segmentierung der Auswahl-Organe in den Volumendaten (VD), wobei
- im Schritt des Erzeugens (S40) die mehreren verschiedenen Visualisierungsbilder (VB) zusätzlich basierend auf der Segmentierungsmaske erzeugt werden.

10. Verfahren nach einem der Ansprüche 7 bis 9, bei dem

- die Patienteninformation (PI) eine auf das dreidimensionale Objekt bezogene Annotation (ANN) aufweist, und
- im Schritt des Erzeugens (S40) die Annotation (ANN) in das 3D-Modell eingefügt wird.

11. Verfahren nach einem der vorhergehenden Ansprüche, bei dem

- das 3D-Modell (3DM) in einer Viewing-Applikation (GUI) bereitgestellt wird, die es dem Nutzer ermöglicht, verschiedene Ansichten des 3D-Modells auszuwählen und anzusehen.

12. Verfahren nach einem der vorhergehenden Ansprüche, ferner mit dem Schritt

- Bereitstellen (S33) einer trainierten Funktion (TF), die dazu ausgebildet ist, basierend auf Volumendaten (VD) einen Parametersatz (PS) zur Ansteuerung eines Bildsynthesealgorithmus (BSA) zu ermitteln, welcher Parametersatzes (PS) geeignet ist, mit dem Bildsynthesealgorithmus (BSA) einen Satz (S) mehrerer verschiedener Visualisierungsbilder (VB) basierend auf dem Volumendatensatz (VD) zu erzeugen, wobei

- im Schritt des Ermittelns (S30) des Parametersatzes (PS) der Parametersatzes (PS) durch Anwenden der trainierten Funktion (TF) ermittelt wird.

13. Vorrichtung (SERV) zum Bereitstellen eines 3D-Modells (3DM) eines durch Volumendaten (VD) repräsentierten dreidimensionalen Objekts mit einer Recheneinheit (20) und einer Schnittstelle (30), wobei

  - die Schnittstelle (30) dazu ausgebildet ist, die Volumendaten (VD) zu empfangen und das 3D-Modell (3DM) bereitzustellen, und
  - die Recheneinheit (20) dazu ausgebildet ist:

    - einen Bildsynthesealgorithmus (BSA) zu hosten, welcher Bildsynthesealgorithmus (BSA) zur Visualisierung des dreidimensionalen Objekts durch Abbilden der Volumendaten (VD) auf Visualisierungspixel (VP) eines zweidimensionalen Visualisierungsbildes (VB) ausgebildet ist,
    - einen Parametersatz (PS) zur Ansteuerung des Bildsynthesealgorithmus (BSA) zu ermitteln, welcher Parametersatz (PS) geeignet ist, mit dem Bildsynthesealgorithmus (BSA) einen Satz (S) mehrerer verschiedener Visualisierungsbilder (VB) basierend auf dem Volumendatensatz (VD) zu erzeugen,
    - den Satz (S) mehrerer verschiedener Visualisierungsbilder (VB) durch Abbilden der Volumendaten (VD) mit dem Bildsynthesealgorithmus (BSA) unter Verwendung des Parametersatzes (PS) zu erzeugen, und
    - das 3D-Modell (3DM) basierend auf dem Satz (S) mehrerer Visualisierungsbilder (VB) zu berechnen.

14. Computerprogrammprodukt mit einem Computerprogramm, welches direkt in einen Speicher einer Vorrichtung (SERV) zum Bereitstellen eines 3D-Modells (3DM) ladbar ist, mit Programmabschnitten, um alle Schritte des Verfahrens zum Bereitstellen eines 3D-Modells nach einem der Ansprüche 1 bis 12 auszuführen, wenn die Programmabschnitte von der Vorrichtung (SERV) ausgeführt werden.

15. Computerlesbares Speichermedium, auf welchem von einer Vorrichtung (SERV) zum Bereitstellen eines 3D-Modells (3DM) lesbare und ausführbare Programmabschnitte gespeichert sind, um alle Schritte des Verfahrens zum Bereitstellen eines 3D-Modells nach einem der Ansprüche 1 bis 12 auszuführen, wenn die Programmabschnitte von der Vorrichtung (SERV) ausgeführt werden.

FIG 1

CLNT

3DM

GUI

30

40

21

22

23

20

SERV

EP 4 293 626 A1

FIG 2

```
┌─────────────┐
│             │──── S10
└─────────────┘
        │
        │   ┌──────┐
        │   │      │──── S15
        │   └──────┘
        ▼
┌─────────────┐
│             │──── S20
└─────────────┘
        │
        ▼
┌─────────────┐
│             │──── S30
└─────────────┘
        │
┌─────┐ │   ┌──────┐
S34 ──│     │ │   │      │──── S31
└─────┘ │   └──────┘
        │   ┌──────┐
        │   │      │──── S32
        │   └──────┘
        │   ┌──────┐
        │   │      │──── S33
        │   └──────┘
        ▼
┌─────────────┐
│             │──── S40
└─────────────┘
        │
        ▼
┌─────────────┐
│             │──── S50
└─────────────┘
        │
        ▼
┌─────────────┐
│             │──── S60
└─────────────┘
```

FIG 3

# FIG 4

FIG 5

230

290

210

240

220

250

200

FIG 6

T10

T20

T30

T40

T50

T60

EUROPÄISCHER RECHERCHENBERICHT

**Nummer der Anmeldung**

EP 22 17 9088

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2017/106597 A1 (GRITZKY ARTHUR [AT] ET AL) 20. April 2017 (2017-04-20) | 1-11, 13-15 | INV. G06T15/08 |
| Y | * Abbildungen 1-4 * <br> * Absätze [0013], [0034], [0036] * <br> * Absatz [0040] – Absatz [0049] * <br> * Absatz [0059] – Absatz [0061] * | 12 | |
| | ----- | | |
| X | US 2021/150811 A1 (LE BERRE YANNICK [FR] ET AL) 20. Mai 2021 (2021-05-20) | 1-11, 13-15 | |
| A | * Abbildungen 1A-3 * <br> * Absätze [0005], [0012] * <br> * Absatz [0018] – Absatz [0035] * <br> * Absätze [0045], [0049] * | 12 | |
| | ----- | | |
| X | US 2015/138201 A1 (BROWN KENNETH [US]) 21. Mai 2015 (2015-05-21) | 1-11, 13-15 | |
| A | * Abbildungen 1A-4 * <br> * Absatz [0008] – Absatz [0011] * <br> * Absätze [0027], [0029], [0031] * <br> * Absatz [0032] * | 12 | |
| | ----- | | **RECHERCHIERTE SACHGEBIETE (IPC)** |
| X | PEI YUCHEN ET AL: "3D Structure Surface Modelling From Volumetric CT Images", 2019 IEEE 16TH INTERNATIONAL SYMPOSIUM ON BIOMEDICAL IMAGING (ISBI 2019), IEEE, 8. April 2019 (2019-04-08), Seiten 476-479, XP033576565, DOI: 10.1109/ISBI.2019.8759432 [gefunden am 2019-07-10] | 1-11, 13-15 | G06T |
| A | * Abbildungen 1-5 * <br> * Zusammenfassung * <br> * Kapitel: 1. "Introduction"-2. "Methods"; Seite 476 – Seite 478 * <br> * Kapitel: 4. "Conclusions"; Seite 479 * | 12 | |
| | ----- <br> -/-- | | |

Der vorliegende Recherchenbericht wurde für alle Patentansprüche erstellt

| Recherchenort | Abschlußdatum der Recherche | Prüfer |
|---|---|---|
| Berlin | 17. November 2022 | Höller, Helmut |

EPO FORM 1503 03.82 (P04C03)

Seite 1 von 2

Europäisches Patentamt
European Patent Office
Office européen des brevets

# EUROPÄISCHER RECHERCHENBERICHT

Nummer der Anmeldung

EP 22 17 9088

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2021/208567 A1 (KNOPLIOCH JEROME [FR] ET AL) 8. Juli 2021 (2021-07-08) | 1-11, 13-15 | |
| A | * Abbildungen 1A-4B *<br>* Absätze [0006], [0016] *<br>* Absatz [0024] – Absatz [0029] *<br>* Absätze [0063], [0064], [0068] *<br>* Absatz [0074] – Absatz [0086] *<br>----- | 12 | |
| X | FENG DONG ET AL: "Interacivie visualisation of a distributed library of pathological human organs", 19991118, 18. November 1999 (1999-11-18), Seiten 17/1-17/5, XP006502626, | 1,5, 13-15 | |
| A | * Kapitel: 2."System Overview"-3. "Graphics Considerations"; Seite 17/2 – Seite 17/4 *<br>----- | 2-4,6-12 | |
| Y | MATTHEW BERGER ET AL: "A Generative Model for Volume Rendering", ARXIV.ORG, CORNELL UNIVERSITY LIBRARY, 201 OLIN LIBRARY CORNELL UNIVERSITY ITHACA, NY 14853, 26. Oktober 2017 (2017-10-26), XP081435113, DOI: 10.1109/TVCG.2018.2816059 * Abbildungen 1, 2, 6 * * Zusammenfassung *<br>----- | 12 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| Berlin | 17. November 2022 | Höller, Helmut |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

Seite 2 von 2

Keskera

**EP 4 293 626 A1**

**ANHANG ZUM EUROPÄISCHEN RECHERCHENBERICHT
ÜBER DIE EUROPÄISCHE PATENTANMELDUNG NR.**

EP 22 17 9088

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-11-2022

| Im Recherchenbericht angeführtes Patentdokument | Datum der Veröffentlichung | Mitglied(er) der Patentfamilie | Datum der Veröffentlichung |
|---|---|---|---|
| US 2017106597 A1 | 20-04-2017 | US 2017106597 A1<br>US 2019365353 A1 | 20-04-2017<br>05-12-2019 |
| US 2021150811 A1 | 20-05-2021 | CN 114616594 A<br>EP 4062378 A2<br>US 2021150811 A1<br>WO 2021101721 A2 | 10-06-2022<br>28-09-2022<br>20-05-2021<br>27-05-2021 |
| US 2015138201 A1 | 21-05-2015 | EP 3072111 A2<br>JP 2017503236 A<br>KR 20160088902 A<br>US 2015138201 A1<br>WO 2015077314 A2 | 28-09-2016<br>26-01-2017<br>26-07-2016<br>21-05-2015<br>28-05-2015 |
| US 2021208567 A1 | 08-07-2021 | CN 114902288 A<br>US 2021208567 A1<br>WO 2021141717 A1 | 12-08-2022<br>08-07-2021<br>15-07-2021 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- EP 3178068 B1

**In der Beschreibung aufgeführte Nicht-Patentliteratur**

- **LEVOY.** Display of Surfaces from Volume Data. *IEEE Computer Graphics and Applications, Ausgabe,* Mai 1988, vol. 8 (3), 29-37

- **KAJIYA.** The rendering equation. *ACM SIGGRAPH Computer Graphics,* 1986, vol. 20 (4), 143-150